(19) Europäisches Patentamt
European Patent Office
Office européen des brevets

(11) Veröffentlichungsnummer: **0 353 641 B1**

(12) # EUROPÄISCHE PATENTSCHRIFT

(45) Veröffentlichungstag der Patentschrift: **07.12.94**

(51) Int. Cl.$^{5}$: **C07D 239/42**

(21) Anmeldenummer: **89113918.0**

(22) Anmeldetag: **28.07.89**

Die Akte enthält technische Angaben, die nach dem Eingang der Anmeldung eingereicht wurden und die nicht in dieser Patentschrift enthalten sind.

(54) **Heterozyklisch substituierte Sulfonylharnstoffe, Verfahren zu ihrer Herstellung und ihre Verwendung als Herbizide oder Pflanzenwachstumsregulatoren.**

(30) Priorität: **05.08.88 DE 3826609**

(43) Veröffentlichungstag der Anmeldung:
**07.02.90 Patentblatt 90/06**

(45) Bekanntmachung des Hinweises auf die Patenterteilung:
**07.12.94 Patentblatt 94/49**

(84) Benannte Vertragsstaaten:
**AT BE CH DE ES FR GB GR IT LI NL**

(56) Entgegenhaltungen:
**EP-A- 0 131 258**
**EP-A- 0 139 947**

(73) Patentinhaber: **Hoechst Schering AgrEvo GmbH**
**Gerichtstrasse 27**
**D-13342 Berlin (DE)**

(72) Erfinder: **Löher, Heinz-Josef, Dr.**
**Amselweg 9**
**D-6238 Hofheim am Taunus (DE)**
Erfinder: **Willms, Lothar, Dr.**
**Schulstrasse 3**
**D-5411 Hillscheid (DE)**
Erfinder: **Frey, Michael, Dr.**
**Meraner Strasse 26a**
**D-8902 Neusäss (DE)**
Erfinder: **Bauer, Klaus, Dr.**
**Doorner Strasse 53 D**
**D-6450 Hanau (DE)**
Erfinder: **Bieringer, Hermann**
**Eichenweg 26**
**D-6239 Eppstein/Taunus (DE)**

**EP 0 353 641 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß heterocyclisch substituierte Alkylsulfonylharnstoffe herbizide und pflanzenwachstumsregulierende Eigenschaften aufweisen (s. EP-A 061661, EP-A 071958, EP-A 131258, DE-OS 3243533). Diese weisen jedoch zum Teil bei ihrer Anwendung Nachteile auf, wie beispielsweise eine hohe Persistenz oder unzureichende Selektivität in wichtigen Nutzkulturen.

Es wurden nun neue heterocyclische Sulfonylharnstoffe mit vorteilhaften herbiziden Eigenschaften gefunden.

Gegenstand der vorliegenden Erfindung sind Verbindungen der allgemeinen Formel (I)

$$R^1-Y-A-NR^2-SO_2-NH-\overset{\overset{\displaystyle Z}{\|}}{C}-NR^3R^4 \qquad (I)$$

worin

A — einen gesättigten oder ungesättigten, unverzweigten oder verzweigten $C_1$-$C_{10}$-Kohlenwasserstoffrest, vorzugsweise ein Rest der Formel $CH_2$, $CH_2CH_2$, CHR, CRR′, $CH_2CHR$ oder $CH_2CRR'$, wobei R und R′ unabhängig voneinander $C_1$-$C_4$-Alkyl oder $C_2$-$C_4$-Alkenyl bedeuten,

$R^1$ — $C_1$-$C_8$-Alkyl, $C_2$-$C_8$-Alkenyl, $C_2$-$C_8$-Alkinyl, $C_3$-$C_8$-Cycloalkyl, $C_5$-$C_8$-Cycloalkenyl oder einen der vorstehenden fünf Reste, der ein- oder mehrfach durch Halogen oder durch solche Reste substituiert ist, die aus der Gruppe aus $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfinyl, $C_1$-$C_6$-Alkylsulfonyl, $C_3$-$C_6$-Cycloalkyl, einem Rest eines drei- bis sechsgliedrigen gesättigten Heterozyklus mit einem Sauerstoffatom im Ring, Furyl, Phenyl und einem Phenylrest, der ein- oder mehrfach durch Reste aus der Gruppe aus Halogen, $C_1$-$C_4$-Alkyl, $C_1$-$C_4$-Alkoxy, $CF_3$, ($C_1$-$C_4$-Alkoxy)-carbonyl und Nitro substituiert ist, ausgewählt sind, oder

$R^1$ — Phenyl oder einen Phenylrest, der ein- oder mehrfach durch Reste aus der Gruppe bestehend aus Halogen, $C_1$-$C_4$-Alkyl, $C_1$-$C_4$-Alkoxy, $CF_3$, ($C_1$-$C_4$-Alkoxy)-carbonyl und Nitro substituiert ist, oder

$R^1$ — einen Rest der Formel

$-CO-R^5$, $-CHR^6-COOR^7$, $-CHR^6-CH_2-COOR^7$ oder $-CH_2-CHR^6-COOR^7$,

wobei in den Formeln $R^5$ für $C_1$-$C_6$-Alkyl, $R^6$ für Wasserstoff, $C_1$-$C_4$-Alkyl, Phenyl oder Benzyl und $R^7$ für H, $C_1$-$C_4$-Alkyl, Phenyl oder Benzyl stehen,

Y — S oder $SO_2$,

$R^2$ — H, $C_1$-$C_8$-Alkyl, $C_2$-$C_8$-Alkenyl, $C_2$-$C_8$-Alkinyl oder einen der vorstehenden drei Reste, der ein- oder mehrfach durch Halogen oder durch Reste aus der Gruppe aus $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfinyl, $c_1$-$c_6$-Alkylsulfonyl, ($C_1$-$C_6$-Alkoxy)-carbonyl, Phenoxycarbonyl, Benzyloxycarbonyl und Phenyl substituiert ist,
$C_3$-$C_8$-Cycloalkyl, das unsubstituiert ist oder ein- oder mehrfach durch Halogen oder ein- oder zweifach durch $C_1$-$C_4$-Alkoxy oder $C_1$-$C_4$-Alkylthio substituiert ist, $C_5$-$C_8$-Cycloalkenyl, Cyclopropylmethyl, Epoxypropyl, Furfuryl, Tetrahydrofurfuryl, Phenoxy-$C_1$-$C_6$-alkyl, Phenyl oder einen der letzten zwei vorstehenden Reste, der im Phenylring durch Halogen, $C_1$-$C_4$-Alkyl, $C_1$-$C_4$-Alkoxy oder Nitro substituiert ist,

$R^3$ — H, $C_1$-$C_8$-Alkyl, $C_2$-$C_8$-Alkenyl, $C_2$-$C_8$-Alkinyl oder $C_1$-$C_4$-Alkoxy,

$R^4$ — einen Rest der Formel

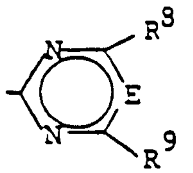 , 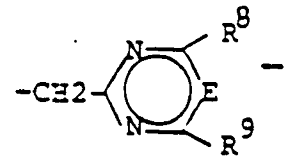 , 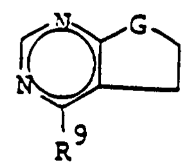 ,

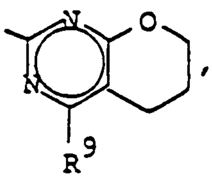 , 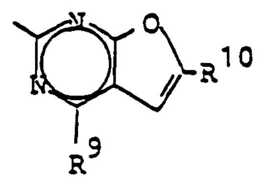 , 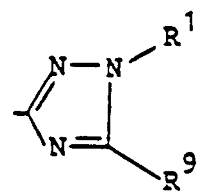 ,

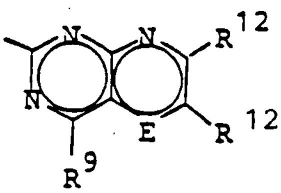 oder 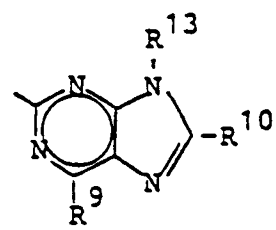 .

| | |
|---|---|
| $R^8$ und $R^9$ | unabhängig voneinander H, Halogen, $C_1$-$C_6$-Alkyl, $C_1$-$C_6$-Alkoxy, $C_1$-$C_6$-Alkylthio oder einen der letzten drei vorstehenden Reste, der ein- oder mehrfach durch Halogen oder ein- oder zweifach durch $C_1$-$C_4$-Alkoxy oder $C_1$-$C_4$-Alkylthio substituiert ist, oder einen Rest $NR^{14}R^{15}$, $C_3$-$C_6$-Cycloalkyl, $-OCHR^{16}COOR^{17}$, $C_3$-$C_5$-Alkenyl, $C_2$-$C_4$-Alkinyl, $C_3$-$C_5$-Alkenyloxy oder $C_3$-$C_5$-Alkinyloxy, |
| $R^{10}$ | Wasserstoff oder $C_1$-$C_4$-Alkyl, |
| $R^{11}$ | $C_1$-$C_4$-Alkyl, $-CHF_2$ oder-$CH_2CF_3$, |
| $R^{12}$ | unabhängig voneinander H, $C_1$-$C_4$-Alkyl, $C_1$-$C_4$-Alkoxy oder Halogen, |
| $R^{13}$ | Wasserstoff, $C_1$-$C_4$-Alkyl, $CHF_2$ oder $CH_2CF_3$, |
| $R^{14}$ und $R^{15}$ | unabhängig voneinander H, $C_1$-$C_4$-Alkyl, $C_2$-$C_4$-Alkenyl oder $C_3$-$C_4$-Alkinyl, |
| $R^{16}$ | Wasserstoff oder $C_1$-$C_4$-Alkyl, |
| $R^{17}$ | Wasserstoff oder $C_1$-$C_4$-Alkyl, |
| E | CH oder N, |
| G | $CH_2$ oder O und |
| Z | O oder S bedeuten, sowie ihre Salze. |

Die Verbindungen der Formel (I) können Salze bilden, bei denen der Wasserstoff der $-SO_2$-NH-Gruppe durch ein für die Landwirtschaft geeignetes Kation ersetzt wird. Diese Salze sind beispielsweise Metallsalze, insbesondere Alkali- und Erdalkalimetallsalze sowie gegebenenfalls alkylierte Ammonium- oder organische Aminsalze. Sie werden vorzugsweise in unter den Reaktionsbedingungen inerten Lösungsmitteln, wie Wasser, Methanol oder Aceton, bei Temperaturen von 0 bis 100 °C aus den Verbindungen der Formel (I) hergestellt. Geeignete Basen zur Herstellung der erfindungsgemäßen Salze sind beispielsweise Alkalicarbonate, wir Kaliumcarbonat, sowie Alkali- und Erdalkalihydroxide, sowie Ammoniak und Ethanolamin.

Von besonderem Interesse sind erfindungsgemäße Verbindungen der Formel (I), in der

$R^1$ $C_1$-$C_4$-Alkyl, einen $C_1$-$C_4$-Alkylrest, der ein- oder mehrfach durch Halogen oder ein- oder zweifach durch $C_1$-$C_4$-Alkoxy, $C_2$-$C_3$-Alkenyloxy, $C_2$-$C_3$-Alkinyloxy, $C_1$-$C_4$-Alkylthio, $C_1$-$C_4$-Alkylsulfinyl, $C_1$-$C_4$-Alkylsulfonyl, Phenyl oder einen Phenylrest, der einbis dreifach durch Reste aus der Gruppe aus Halogen, $C_1$-$C_4$-Alkyl, $C_1$-$C_4$-Alkoxy, $CF_3$, ($C_1$-$C_4$-Alkoxy)-carbonyl und Nitro substituiert ist, substituiert ist, oder $C_3$-$C_8$-Cycloalkyl oder ein $C_3$-$C_8$-Cycloalkylrest, der ein- oder mehrfach durch Halogen oder ein- bis zweifach durch $C_1$-$C_4$-Alkoxy oder $C_1$-$C_4$-Alkylthio substituiert ist, oder $C_5$-$C_8$-Cycloalkenyl, Cyclopropylmethyl, Epoxypropyl, Furfuryl, Tetrahydrofurfuryl, Benzyl, Phenyl, oder einen Benzyl- oder Phenylrest, der im Phenylring durch einen oder mehrere Reste aus der

Gruppe aus Halogen, $C_1$-$C_4$-Alkyl, $C_1$-$C_4$-Alkoxy, $CF_3$, ($C_1$-$C_4$-Alkoxy)-carbonyl und Nitro substituiert ist, oder
einen Rest der Formel
-$COR^5$, $CHR^6$-$COOR^7$, $CHR^6$-$CH_2$-$COOR^7$ oder $CH_2$-$CHR^6$-$COOR^7$,

$R^5$ $C_1$-$C_4$-Alkyl,
$R^6$ H, $C_1$-$C_4$-Alkyl, Benzyl oder Phenyl und
$R^7$ H, $C_1$-$C_4$-Alkyl, Benzyl oder Phenyl bedeuten.

Von besonderem Interesse sind erfindungsgemäße Verbindungen der Formel (I), in der

$R^2$ $C_1$-$C_4$-Alkyl, einen $C_1$-$C_4$-Alkylrest, der ein- oder mehrfach durch Halogen oder ein- oder zweifach durch $C_1$-$C_4$-Alkoxy, $C_2$-$C_4$-Alkenyloxy, Propargyloxy, $C_1$-$C_4$-Alkylthio, $C_1$-$C_4$-Alkylsulfinyl, $C_1$-$C_4$-Alkylsulfonyl, ($C_1$-$C_4$-Alkoxy)-carbonyl, Phenoxycarbonyl, Benzyloxycarbonyl oder Phenyl substituiert ist, oder $C_3$-$C_8$-Cycloalkyl bedeutet.

Von besonderem Interesse sind weiterhin erfindungsgemäße Verbindungen der Formel (I), in der

$R^4$ einen Rest der Formel

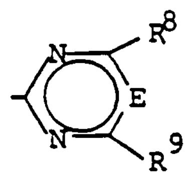 ,

und

$R^8$ und $R^9$ unabhängig voneinander Halogen, $C_1$-$C_4$-Alkyl, $C_1$-$C_4$-Alkoxy, $C_1$-$C_4$-Alkylthio oder einen der letzten drei vorstehenden Reste, die ein- oder mehrfach durch Halogen oder ein- oder zweifach durch $C_1$-$C_4$-Alkoxy, $C_1$-$C_4$-Alkylthio substituiert sind, oder einen Rest $NR^{14}R^{15}$, $C_3$-$C_6$-Cycloalkyl, -$OCHR^{16}$ $COOR^{17}$, Allyl, Propargyl, Allyloxy oder Propargyloxy bedeuten,
$R^{14}$ und $R^{15}$ unabhängig voneinander H oder $C_1$-$C_4$-Alkyl,
$R^{16}$ H oder $C_1$-$C_4$-Alkyl,
$R^{17}$ $C_1$-$C_4$-Alkyl und
E CH oder N bedeuten.

Bevorzugte Verbindungen der Formel (I) sind solche, bei denen

A einen Rest der Formel -$CH_2$- oder -$CH_2$-$CH_2$-,
$R^1$ $C_1$-$C_4$-Alkyl, einen $C_1$-$C_4$-Alkylrest, der ein- oder mehrfach durch Halogen oder ein- bis zweifach durch $C_1$-$C_4$-Alkoxy substituiert ist,
$C_3$-$C_8$-Cycloalkyl, das ein- oder mehrfach durch Halogen substituiert ist oder unsubstituiert ist,
Benzyl, Phenyl oder einen Benzyl- oder Phenylrest, der im Phenylring ein- oder mehrfach durch Halogen, $C_1$-$C_4$-Alkyl, $C_1$-$C_4$-Alkoxy, $CF_3$, ($C_1$-$C_4$-Alkoxy)-carbonyl oder Nitro substituiert ist oder
einen Rest der Formel

-$CR^6H$-$CO_2R^7$

worin $R^6$ und $R^7$ gleich oder verschieden sind und jeweils H, $C_1$-$C_4$-Alkyl, Phenyl oder Benzyl bedeuten,
$R^2$ $C_1$-$C_4$-Alkyl, das unsubstituiert ist oder ein- oder mehrfach durch Halogen oder durch ($C_1$-$C_4$-Alkoxyl)-carbonyl, Phenyloxycarbonyl, Benzyloxycarbonyl oder Phenyl substituiert ist,
$R^3$ H, $C_1$-$C_4$-Alkyl oder Allyl, insbesondere H,
$R^4$ einen Rest der Formel

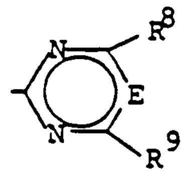 ,

$R^8$ und $R^9$ unabhängig voneinander Halogen, $C_1$-$C_4$-Alkyl, $C_1$-$C_4$-Alkoxy oder einen der letzten beiden vorstehenden Reste, der halogeniert ist, insbesondere die Reste $CH_3$, $OCH_3$, $OC_2H_5$, Cl, $OCF_2H$, $CF_3$,

E CH oder N und

Z O oder S bedeuten.

Ein weiterer Gegenstand der vorliegenden Erfindung ist das Verfahren zur Herstellung von Verbindungen der allgemeinen Formel (I) oder deren Salzen, dadurch gekennzeichnet, daß man

(a) eine Verbindung der Formel (II)

$R^1$-Y-A-$NR^2$-$SO_2$-N = C = Z (II),

mit einer Verbindung der Formel (III)

H-$NR^3R^4$ (III),

umsetzt, wobei in den Formeln (II) und (III) A, Y, Z, $R^1$, $R^2$, $R^3$ und $R^4$ die bei Formel (I) angegebenen Bedeutungen haben, oder

(b) eine Verbindung der Formel (IV)

$R^1$-Y-A-$NR^2$-$SO_2$-$NH_2$ (IV)

mit einem Carbamat bzw. Thiocarbamat der Formel (V)

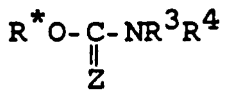 (V)

umsetzt,

wobei in den Formeln (IV) und (V) $R^1$, $R^2$, $R^3$, $R^4$, A, Y und Z die bei Formel (I) angegebenen Bedeutungen haben und $R^*C_1$-$C_6$-Alkyl, $C_1$-$C_4$-Halogenalkyl, Phenyl oder einen Phenylrest bedeutet, der ein- oder mehrfach durch Halogen, $C_1$-$C_4$-Alkyl oder Nitro substituiert ist, oder

(c) ein Carbamat bzw. Thiocarbamat der Formel (VI) mit einer Verbindung der oben genannten Formel (III)

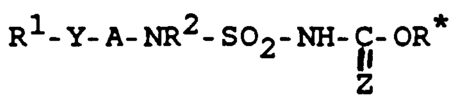 (VI)

umsetzt, wobei $R^1$, $R^2$, $R^*$, Y, A und Z die genannten Bedeutungen haben oder

(d) eine Verbindung der Formel (VII) oder (VIII)

$R^1$-Y-A-NH-$R^2$ (VII)

$R^1$-Y-A-NH-$R^2$ x HCl (VIII)

mit einer Verbindung der Formel (IX)

$$ClSO_2\text{-}NH\text{-}\underset{\underset{Z}{\|}}{C}\text{-}NR^3R^4 \qquad (IX)$$

umsetzt, wobei in den Formeln (VII) bis (IX) A, Y, Z, $R^1$, $R^2$, $R^3$ und $R^4$ die oben angegebenen Bedeutungen haben.

Die Umsetzung der Verbindungen der Formel (II) und (III) erfolgt vorzugsweise in unter den Reaktionsbedingungen inerten aprotischen Lösungsmitteln, wie beispielsweise Acetonitril, Dichlormethan, Toluol, Tetrahydrofuran oder Dioxan bei Temperaturen von 0 °C bis Siedetemperatur des Reaktionsgemischs. Die Alkylsulfonylisocyanate bzw. -isothiocyanate der Formel (II) lassen sich analog üblichen Verfahrensweisen aus den entsprechenden Sulfonamiden der obengenannten Formel (IV) in einfacher Weise herstellen (vgl. z.B. EP-A 085276).

Die Ausgangsstoffe der Formel (III) sind bekannt oder lassen sich nach im Prinzip bekannten Verfahrensweisen herstellen, z.B. durch Cyclisierung entsprechender Guanidinderivate mit entsprechend substituierten 1,3-Diketonen; vgl. z.B. "The chemistry of heterocylic compounds" Vol. XVI (1962) and Supplement I (1970). Eine andere Möglichkeit besteht in der Derivatisierung von Cyanurchlorid; vgl. z.B. "The chemistry of heterocyclic compounds" L. Rapaport: "s-Triazines and Derivatives" (1959).

Die Umsetzung einer Verbindung (IV) mit einem heterocyclischen Carbamat der Formel (V) wird vorzugsweise in Gegenwart von tertiären organischen Basen, beispielsweise 1,8-Diazabicyclo-[5,4,0]-undec-7-en (DBU), in inerten Lösungsmitteln wie Acetonitril oder Dioxan bei einer Temperatur von 20 °C bis zur Siedetemperatur des Reaktionsgemischs durchgeführt; das Verfahren ist analog dem entsprechenden Verfahren aus EP-A 44807. Die hierzu erforderlichen Carbamate (V) sind literaturbekannt oder werden analog bekannten oder an sich üblichen Verfahren hergestellt; ein entsprechendes Verfahren ist in EP-A 70804 beschrieben.

Die Carbamate der Formel (VI) sind neu und lassen sich durch Umsetzung der Verbindungen der Formel (IV) mit entsprechenden Chlorameisensäureestern herstellen (vgl. EP-A 87780). Die Umsetzung der Carbamate bzw. Thiocarbamate der Formel (VI) mit den Aminoheterocyclen der Formel (III) führt man vorzugsweise in inerten Lösungsmitteln, beispielsweise Toluol, Xylol, Chlorbenzol, Dioxan und Acetonitril, bei einer Temperatur von 20 °C bis zur Siedetemperatur der betreffenden Reaktionsmischung durch.

Die Verbindung der Formel (VII) lassen sich mit Basen aus Verbindungen der Formel (VIII) herstellen. Verbindungen der Formeln (VIII) und (IX) lassen sich analog literaturbekannten oder an sich üblichen Verfahren herstellen (vgl. US-A 4,016,266 und J. Heterocycl. Chem. 8, 597 (1971)).

Die Sulfonylharnstoffe der Formel (I), welche in den aliphatischen Resten A, $R^1$, $R^2$ ein oder mehrere asymmetrische Kohlenstoffatome enthalten, liegen in enantiomeren und/oder diastereomeren Formen vor. Im allgemeinen werden die entsprechenden erfindungsgemäßen Verbindungen als Racemate oder als diastereomeren Gemische erhalten. Falls gewünscht, können die üblichen Techniken zur Trennung dieser Gemische in die sterisch einheitlichen Bestandteile angewendet werden. Auch durch Verwendung von sterisch einheitlichen Ausgangsmaterialien ist eine Reindarstellung der genannten Verbindungen möglich.

Die Formel (I) umfaßt daher alle oben genannten enantiomeren und diastereomeren Formen der oben definierten Verbindungen.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B.. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend

bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltstoffen und zur Ernteerleichterung wie z.B. durch Auslösen von Desikkation und Wuchstauchung eingesetzt werden. Desweiteren eignen se sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden.

Gegenstand der Erfindung sind daher auch herbizide bzw. wachstumsregulierende Mittel, die gekennzeichnet sind durch einen Gehalt an einer Verbindung der Formel I in Kombination mit üblichen Formulierungshilfsmitteln und Inertstoffen, sowie deren Verwendung im landwirtschaftlichen Bereich.

Die Spritzpulver sind in Wasser gleichmäßig dispergierbare Präparate, die neben dem Wirkstoff außer gegebenenfalls einem Verdünnungs- oder Inertstoff noch Netzmittel, z.B. polyoxyethylierte Alkylphenole, polyoxethylierte Fettalkohole, Alkyl- oder Alkylphenylsulfonate und Dispergiermittel, z.B. ligninsulfonsaures Natrium, 2,2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielswiese verwendet werden:
Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrat mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

Die erfindungsgemäßen Mittel enthalten die Wirkstoffe der Formel (I) in der Regel in einer Menge von 2 bis 90 Gew.-%.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel,

Füllstoffe usw. verwendet werden.

Daneben enthalten die genannten Wirkstofformulierungen gegebenenfalls die jeweils üblichen Haft-, Netz-, Dispergier-, Emulgier-, Penetrations- oder Lösungsmittel oder Füll- oder Trägerstoffe.

Diese oben genannten Formulierungstypen werden beispielsweise beschrieben in: Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die für diese Formulierungen zu verwendenden Formulierungshilfsmittel (Inertmaterialien, Emulgatoren, Netzmittel, Tenside, Lösungsmittel etc.) sind beispielsweise in Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood oder "Encyclopedia of Surface Active Agents", Chem. Publ. Co. Inc., N.Y. 1964 beschrieben.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden, sind gegebenenfalls möglich.

Die Erfindung wird durch nachstehende Beispiele näher erläutert.

**Formulierungsbeispiele**

A. Ein Stäubemittel wird erhalten, indem man 10 Gewichtsteile Wirkstoff und 90 Gewichtsteile Talkum oder Inertstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (®Triton X 207), 3 Gewichtsteilen Isotridecanolpolyglykolether mit im Mittel 8 Ethylenoxyeinheiten (EO) und 71 Gewichtsteilen paraffinischem Mineralöl (Siedebereich z.B. ca, 255 bis über 377 °C) mischt und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 EO) als Emulgator.

**Chemische Beispiele**

**N-(β-Ethylsulfonylethyl)-n-butylamin**

Zu einer Lösung von 73 g n-Butylamin und 10,1 g Triethylamin in 100 ml Toluol wird bei 50 °C Innentemperatur eine Lösung von 15,7 g Chlorethylethylsulfon in 50 ml Toluol innerhalb von 10 min zugetropft. Man rührt 5 h bei 50 °C nach, läßt auf Raumtemperatur abkühlen, saugt den Niederschlag ab und engt das Filtrat am Rotavapor ein. Das zurückbleibende gelbe Öl (18,7 g) wird ohne weitere Reinigung umgesetzt.

**Ethyl-(β-methylaminoethyl)-sulfon**

Zu einer Lösung von 15,7 g Chlorethylethylsulfon in 100 ml Toluol wird bei 50 °C Innentemperatur gasförmiges Methylamin bis zu Sättigung eingeleitet (ca. 30 min). Man rührt 3 h bei 50 °C nach, saugt vom Niederschlag ab und engt am Rotavapor ein. Die zurückbleibende gelbe, leichtbewegliche Flüssigkeit (12,3 g) wird ohne weitere Reinigung umgesetzt.

**N-[N-(β-Ethylsulfonylethyl)-N-methyl]sulfamoyl-N′-(4,6-dimethoxypyrimid-2-yl)-harnstoff**
(Beispiel Nr. 235)

Zu einer Lösung von 7,8 g 2-Amino-4,6-dimethoxypyrimidin in 50 ml $CH_2Cl_2$ wird bei -78 °C eine Lösung von 7,1 g Chlorsulfonylisocyanat in 50 ml Methylenchlorid getropft. Man läßt auf -30 °C erwärmen und tropft eine Lösung von 7,6 g Ethyl-(β-methylaminoethyl)-sulfon und 5,1 g Triethylamin in 50 ml Methylenchlorid zu. Man läßt auf Raumtemperatur erwärmen und rührt 16 Stunden nach. Das Reaktionsgemisch wird mit je 100 ml 2-normaler Salzsäure und Wasser gewaschen, über Magnesiumsulfat getrocknet und eingeengt. Verrühren des Rückstands mit Ether ergibt 10,5 g Produkt. Fp. = 150-151 °C.

**Methylmercaptomethyl-methylammoniumchlorid**

20 g Triazin in 350 ml Acetonitril werden auf -30 °C gekühlt, anschließend werden 18,6 g HCl/Gas bei -30 °C eingegast. Es wird 10 min gerührt und anschließend 24 g Methylmercaptan zugegeben. Langsam wird auf Raumtemperatur erwärmt und 24 Stunden stehengelassen. Das Reaktionsgemisch wird einrotiert, der Rückstand mit Methylenchlorid ausgerührt und abgesaugt, wodurch 38,7 g Produkt erhalten werden.

**N-[(4,6-Dimethylpyrimid-2-yl-aminocarbonyl]-N′-(methylmercaptomethyl)-N′-methyl-aminosulfonamid**
(Beispiel Nr. 1)

2,4 ml Chlorsulfonylisocyanat werden in 30 ml Methylenchlorid gelöst und auf -20 °C gekühlt. Dann wird 3,08 g 2-Amino-4,6-dimethylpyrimidin fest zugegeben und 0,5 Stunden nachgerührt. Man läßt auf Raumtemperatur erwärmen, wobei eine klare Lösung entsteht. Dann wird erneut auf -70 °C abgekühlt und eine Mischung aus 6,9 ml Triethylamin und 13,19 g Methylmercaptomethyl-methylammoniumchlorid in 30 ml Methylenchlorid zugetropft. Es wird 0,5 Stunden bei -70 °C und 1 Stunde bei Raumtemperatur nachgerührt. Dann setzt man Wasser zu und extrahiert mit Methylenchlorid. Die org. Phase wird mehrmals mit Acetonitril ausgerührt. So werden 3,2 g Produkt erhalten. Fp. = 138-140 °C.

**N-[(4,6-Dimethyl-pyrimid-2-yl-aminocarbonyl]-N′-(methylsulfonylmethyl)-N′-methyl-aminosulfonamid**
(Beispiel Nr. 12)

3,19 g N-[(4,6-Dimethyl-pyrimid-2-yl-aminocarbonyl]-N′-methylmercaptomethyl-N′-methyl-aminosulfonamid werden in 60 ml Eisessig gelöst. Bei 0 °C werden dazu 100 ml einer 3 %igen $KMnO_4$-Lösung getropft. 2 Stunden wird bei Raumtemperatur nachgerührt. Mit Eiswasser wird dann verdünnt und bis zur Entfärbung Schwefeldioxid eingeleitet. Der anfallende Feststoff wird abgesaugt und mit Wasser gewaschen. Nach Trocknung an der Luft werden 2,2 g Produkt erhalten. Fp. = 146-148 °C.

Analog der vorstehend beschriebenen Beispiele werden die in Tabelle 1 aufgeführten Verbindungen erhalten.

TABELLE 1

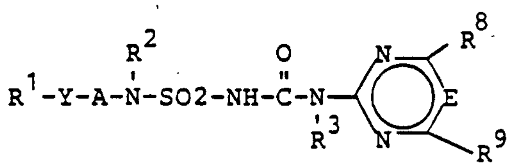

| Bsp. Nr. | A | $-Y-R^1$ | $R^2$ | $R^3$ | $R^8$ | $R^9$ | E | Fp. (°C) |
|---|---|---|---|---|---|---|---|---|
| 1 | $CH_2$ | $-S-CH_3$ | $-CH_3$ | H | $CH_3$ | $CH_3$ | CH | 138-140 |
| 2 | " | " | " | H | $OCH_3$ | $CH_3$ | " | |
| 3 | " | " | " | H | $OCH_3$ | $OCH_3$ | " | 152-154 |
| 4 | " | " | " | H | " | Cl | " | |
| 5 | " | " | " | H | $OCHF_2$ | $CH_3$ | " | |
| 6 | " | " | " | H | " | $CF_3$ | " | |
| 7 | " | " | " | H | " | $OCHF_2$ | " | |
| 8 | " | " | " | H | $CH_3$ | Cl | " | |
| 9 | " | " | " | H | $OCH_3$ | Br | " | |
| 10 | " | " | " | H | " | $NHCH_3$ | " | |
| 11 | " | " | " | H | $OCHF_2$ | $OCH_3$ | " | |
| 12 | " | $-SO_2-CH_3$ | " | H | $CH_3$ | $CH_3$ | " | 146-148 |
| 13 | " | " | " | H | $OCH_3$ | $CH_3$ | " | |
| 14 | " | " | " | H | " | $OCH_3$ | " | 176-178 |
| 15 | " | " | " | H | " | Cl | " | |
| 16 | " | " | " | H | $OCHF_2$ | $CH_3$ | " | |
| 17 | " | " | " | H | " | $CF_2$ | " | |
| 18 | " | " | " | H | " | $OCHF_2$ | " | |
| 19 | " | " | " | H | $CH_3$ | Cl | " | |
| 20 | " | " | " | H | $OCH_3$ | Br | " | |

Forts. Tabelle 1

| Bsp. Nr. | A | $-Y-R^1$ | $R^2$ | $R^3$ | $R^8$ | $R^9$ | E | Fp.(°C) |
|---|---|---|---|---|---|---|---|---|
| 21 | $CH_2$ | $-SO_2-CH_3$ | $-CH_3$ | H | $OCH_3$ | $NHCH_3$ | CH | |
| 22 | " | " | " | H | $OCHF_2$ | $OCH_3$ | " | |
| 23 | " | " | " | H | $CH_3$ | $CH_3$ | " | 140-142 Z. |
| 24 | " | " | " | H | $OCH_3$ | $CH_3$ | " | |
| 25 | " | " | " | H | " | $OCH_3$ | " | 135-138 Z. |
| 26 | " | " | " | H | " | Cl | " | |
| 27 | " | " | " | H | $OCHF_2$ | $CH_3$ | " | |
| 28 | " | " | " | H | " | $CF_3$ | " | |
| 29 | " | " | " | H | " | $OCHF_2$ | " | |
| 30 | " | " | " | H | $CH_3$ | Cl | " | |
| 31 | " | " | " | H | $OCH_3$ | Br | " | |
| 32 | " | " | " | H | " | $NHCH_3$ | " | |
| 33 | " | " | " | H | " | $OCH_3$ | " | |
| 34 | " | $-SO_2-C_2H_5$ | " | H | $CH_3$ | $CH_3$ | " | |
| 35 | " | " | " | H | $OCH_3$ | $CH_3$ | " | |
| 36 | " | " | " | H | " | $OCH_3$ | " | |
| 37 | " | | " | H | " | Cl | " | |
| 38 | " | " | " | H | $OCHF_2$ | $CH_3$ | " | |
| 39 | " | " | " | H | " | $CF_3$ | " | |
| 40 | " | " | " | H | " | $OCHF_2$ | " | |
| 41 | " | " | " | H | $CH_3$ | Cl | " | |
| 42 | " | " | " | H | $OCH_3$ | Br | " | |
| 43 | " | " | " | H | " | $NHCH_3$ | " | |
| 44 | " | " | " | H | $OCHF_2$ | $OCH_3$ | " | |
| 45 | " | $-SO_2-CH_3$ | " | H | $OCH_3$ | $OCH_3$ | N | |

Forts. Tabelle 1

| Bsp. Nr. | A | $-Y-R^1$ | $R^2$ | $R^3$ | $R^8$ | $R^9$ | E | Fp. (°C) |
|---|---|---|---|---|---|---|---|---|
| 46 | $CH_2$ | $-SO_2-CH_3$ | $-CH_3$ | H | $OCHF_2$ | $OCHF_2$ | N | |
| 47 | " | " | " | H | $CH_3$ | $CH_3$ | N | |
| 48 | " | $-SCH_3$ | " | H | $OCH_3$ | $OCH_3$ | N | |
| 49 | " | " | " | H | $CH_3$ | $OCHF_2$ | N | |
| 50 | " | $-S-CH_2-C_6H_5$ | " | H | $CH_3$ | $CH_3$ | CH | 149-152 Z. |
| 51 | " | " | " | H | $OCH_3$ | $CH_3$ | " | |
| 52 | " | " | " | H | $OCH_3$ | $OCH_3$ | " | 114-117 Z. |
| 53 | " | " | " | H | $OCH_3$ | Cl | " | |
| 54 | " | " | " | H | $OCHF_2$ | $CH_3$ | " | |
| 55 | " | " | " | H | $OCHF_2$ | $CF_3$ | " | |
| 56 | " | " | " | H | $OCHF_2$ | $OCHF_2$ | " | |
| 57 | " | " | " | H | $CH_3$ | Cl | " | |
| 58 | " | " | " | H | $OCH_3$ | Br | " | |
| 59 | " | " | " | H | $OCH_3$ | $NHCH_3$ | " | |
| 60 | " | " | " | H | $OCHF_2$ | $OCH_3$ | " | |
| 61 | " | " | $-CH(CH_3)_2$ | H | $CH_3$ | $CH_3$ | " | 136-138 |
| 62 | " | " | " | H | $OCH_3$ | $CH_3$ | " | |
| 63 | " | " | " | H | $OCH_3$ | $OCH_3$ | " | 119-121 |
| 64 | " | " | " | H | $OCH_3$ | Cl | " | |
| 65 | " | " | " | H | $OCHF_2$ | $CH_3$ | " | |
| 66 | " | " | " | H | " | $CF_3$ | " | |
| 67 | " | " | " | H | " | $OCHF_2$ | " | |
| 68 | " | " | " | H | $CH_3$ | Cl | " | |
| 69 | " | " | " | H | $OCH_3$ | Br | " | |
| 70 | " | " | " | H | $OCH_3$ | $NHCH_3$ | " | |
| 71 | " | " | " | H | $OCHF_2$ | $OCH_3$ | " | |

Forts. Tabelle 1

| Bsp. Nr. | A | $-Y-R^1$ | $R^2$ | $R^3$ | $R^8$ | $R^9$ | E | Fp. (°C) |
|---|---|---|---|---|---|---|---|---|
| 72 | $CH_2$ | $-SO_2-CH_3$ | $-CH_3$ | $CH_3$ | $OCH_3$ | $OCH_3$ | N | |
| 73 | " | " | " | " | $CH_3$ | $OCHF_2$ | N | |
| 74 | " | " | " | H | $OCH_3$ | $OCH_3$ | N | |
| 75 | " | " | " | $CH_3$ | $OCH_3$ | $CF_3$ | N | |
| 76 | " | $-S-CH_3$ | " | " | $OCH_3$ | $OCH_3$ | CH | |
| 77 | " | " | " | " | $OCHF_2$ | $OCHF_2$ | CH | |
| 78 | " | " | " | " | $OCH_3$ | Br | " | |
| 79 | " | $-SO_2-CH_2C_6H_5$ | " | H | $CH_3$ | $CH_3$ | " | 132-135 Z. |
| 80 | " | " | " | " | $OCH_3$ | $CH_3$ | " | |
| 81 | " | " | " | " | $OCH_3$ | $OCH_3$ | " | 156-158 Z. |
| 82 | " | " | " | " | $OCH_3$ | Cl | " | |
| 83 | " | " | " | " | $OCHF_2$ | $CH_3$ | " | |
| 84 | " | " | " | " | $OCHF_2$ | $CF_3$ | " | |
| 85 | " | " | " | " | $OCHF_2$ | $OCHF_2$ | " | |
| 86 | " | " | " | " | $CH_3$ | Cl | " | |
| 87 | " | " | " | " | $OCH_3$ | Br | " | |
| 88 | " | " | " | " | $OCH_3$ | $NHCH_3$ | " | |
| 89 | " | " | " | " | $OCHF_2$ | $OCH_3$ | " | |
| 90 | " | $-S-CH_2CO_2CH_3$ | " | " | $CH_3$ | $CH_3$ | " | 124-126 |
| 91 | " | " | " | " | $OCH_3$ | $CH_3$ | " | |
| 92 | " | " | " | " | $OCH_3$ | $OCH_3$ | " | 128-131 |
| 93 | " | " | " | " | $OCH_3$ | Cl | " | |
| 94 | " | " | " | " | $OCHF_2$ | $CH_3$ | " | |
| 95 | " | " | " | " | $OCHF_2$ | $CF_3$ | " | |
| 96 | " | " | " | " | $OCHF_2$ | $OCHF_2$ | " | |
| 97 | " | " | " | " | $CH_3$ | Cl | " | |

Forts. Tabelle 1

| Bsp. Nr. | A | -Y-$R^1$ | $R^2$ | $R^3$ | $R^8$ | $R^9$ | E | Fp. (°C) |
|---|---|---|---|---|---|---|---|---|
| 98 | $CH_2$ | $-S-CH_2CO_2CH_3$ | $CH_3$ | H | $OCH_3$ | Br | CH | |
| 99 | " | " | " | " | $OCH_3$ | $NHCH_3$ | " | |
| 100 | " | " | " | " | $OCHF_2$ | $OCH_3$ | " | |
| 101 | " | " | " | $CH_3$ | $OCH_3$ | $OCH_3$ | " | |
| 102 | " | " | " | " | $OCH_3$ | $OCH_3$ | N | |
| 103 | " | " | " | " | $OCH_3$ | $CF_3$ | N | |
| 104 | " | " | " | " | $OCHF_2$ | $OCHF_2$ | CH | |
| 105 | " | " | " | " | $OCH_3$ | Br | " | |
| 106 | " | $-S-CH_3$ | $C_2H_5$ | H | $CH_3$ | $CH_3$ | " | 129-131 |
| 107 | " | " | " | " | $OCH_3$ | $CH_3$ | " | |
| 108 | " | " | " | " | $OCH_3$ | $OCH_3$ | " | 116-118 |
| 109 | " | " | " | " | $OCH_3$ | Cl | " | |
| 110 | " | " | " | " | $OCHF_2$ | $CH_3$ | " | |
| 111 | " | " | " | " | $OCHF_2$ | $CF_3$ | " | |
| 112 | " | " | " | " | $OCHF_2$ | $OCHF_2$ | " | |
| 113 | " | " | " | " | $CH_3$ | Cl | " | |
| 114 | " | " | " | " | $OCH_3$ | Br | " | |
| 115 | " | " | " | " | $OCH_3$ | $NHCH_3$ | " | |
| 116 | " | " | " | " | $OCHF_2$ | $OCH_3$ | " | |
| 117 | " | $-SCOCH_3$ | $-CH(CH_3)_2$ | " | $CH_3$ | $CH_3$ | " | 152-154 Z. |
| 118 | " | " | " | " | $OCH_3$ | $CH_3$ | " | |
| 119 | " | " | " | " | $OCH_3$ | $OCH_3$ | " | 155-147 Z. |
| 120 | " | " | " | " | $OCH_3$ | Cl | " | |
| 121 | " | " | " | " | $OCHF_2$ | $CH_3$ | " | |
| 122 | " | " | " | " | $OCHF_2$ | $CF_3$ | " | |
| 123 | " | " | " | " | " | $OCHF_2$ | " | |

Forts. Tabelle 1

| Bsp. Nr. | A | -Y-R[1] | $R^2$ | $R^3$ | $R^8$ | $R^9$ | E | Fp. (°C) |
|---|---|---|---|---|---|---|---|---|
| 124 | $CH_2$ | $-SCOCH_3$ | $-CH(CH_3)_2$ | H | $CH_3$ | Cl | CH | |
| 125 | " | " | " | " | $OCH_3$ | Br | " | |
| 126 | " | " | " | " | $OCH_3$ | $NHCH_3$ | " | |
| 127 | " | " | " | " | $OCHF_2$ | $OCH_3$ | " | |
| 128 | " | $-SCH_3$ | $-C_2H_5$ | $CH_3$ | $OCH_3$ | $OCH_3$ | " | |
| 129 | " | " | " | " | " | Br | " | |
| 130 | " | " | " | H | $OCH_3$ | $OCH_3$ | N | |
| 131 | " | " | " | " | $OCHF_2$ | $OCHF_2$ | N | |
| 132 | " | " | " | $CH_3$ | $CH_3$ | Cl | CH | |
| 133 | " | $-SCH_2CH_2CO_2CH_3$ | $CH_3$ | H | $CH_3$ | $CH_3$ | " | 134-136 |
| 134 | " | " | " | " | $OCH_3$ | $CH_3$ | " | |
| 185 | " | " | " | " | " | $OCH_3$ | " | 131-133 |
| 136 | " | " | " | " | " | Cl | " | |
| 137 | " | " | " | " | $OCHF_2$ | $CH_3$ | " | |
| 138 | " | " | " | " | " | $CF_3$ | " | |
| 139 | " | " | " | " | " | $OCHF_2$ | " | |
| 140 | " | " | " | " | $CH_3$ | Cl | " | |
| 141 | " | " | " | " | $OCH_3$ | Br | " | |
| 142 | " | " | " | " | $OCH_3$ | $NHCH_3$ | " | |
| 143 | " | " | " | " | $OCHF_2$ | $OCH_3$ | " | |
| 144 | " | $-S-C_6H_4-2-CO_2C_2H_5$ | " | " | $CH_3$ | $CH_3$ | " | 145-147 |
| 145 | " | " | " | " | $OCH_3$ | $CH_3$ | " | |
| 146 | " | " | " | " | $OCH_3$ | $OCH_3$ | " | 118 |
| 147 | " | " | " | " | $OCH_3$ | Cl | " | |
| 148 | " | " | " | " | $OCHF_2$ | $CH_3$ | " | |
| 149 | " | " | " | " | $OCHF_2$ | $CF_3$ | " | |

Forts. Tabelle 1

| Bsp. Nr. | A | -Y-$R^1$ | $R^2$ | $R^3$ | $R^8$ | $R^9$ | E | Fp. (°C) |
|---|---|---|---|---|---|---|---|---|
| 150 | CH2 | $-S-C_6H_4-2-CO_2C_2H_5$ | $CH_3$ | H | $OCHF_2$ | $OCHF_2$ | CH | |
| 151 | " | " | " | " | $CH_3$ | Cl | " | |
| 152 | " | " | " | " | $OCH_3$ | Br | " | |
| 153 | " | " | " | " | $OCH_3$ | $NHCH_3$ | " | |
| 154 | " | " | " | " | $OCHF_2$ | $OCH_3$ | " | |
| 155 | " | " | " | $CH_3$ | $OCH_3$ | $OCH_3$ | " | |
| 156 | " | " | " | " | $CH_3$ | $CH_3$ | " | |
| 157 | " | " | " | H | $OCH_3$ | $OCH_3$ | N | |
| 158 | " | " | " | " | $CH_3$ | $CH_3$ | N | |
| 159 | " | " | " | " | $CH_3$ | $CH_3$ | CH | 138-140 |
| 160 | " | " | " | H | $OCH_3$ | $CH_3$ | CH | |
| 161 | " | " | " | H | $OCH_3$ | $OCH_3$ | CH | 130 |
| 162 | " | " | " | H | $OCH_3$ | Cl | CH | |
| 163 | " | " | " | H | $OCHF_2$ | $CH_3$ | " | |
| 164 | " | " | " | " | $OCHF_2$ | $CF_3$ | " | |
| 165 | " | " | " | " | $OCHF_2$ | $OCHF_2$ | " | |
| 166 | " | " | " | " | $CH_3$ | Cl | " | |
| 167 | " | " | " | " | $OCH_3$ | Br | " | |
| 168 | " | " | " | " | $OCH_3$ | $NHCH_3$ | " | |
| 169 | " | " | " | " | $OCHF_2$ | $OCH_3$ | " | |
| 170 | " | $-SCH_3$ | $-CH_2CH_2CH_3$ | " | $CH_3$ | $CH_3$ | " | 128-131 |
| 171 | " | " | " | " | $OCH_3$ | $CH_3$ | " | |
| 172 | " | " | " | " | $OCH_3$ | $OCH_3$ | " | 142-14[illegible] |
| 173 | " | " | " | " | $OCH_3$ | Cl | " | |
| 174 | " | " | " | " | $OCHF_2$ | $CH_3$ | " | |
| 175 | " | " | " | " | $OCHF_2$ | $CF_3$ | " | |

Forts. Tabelle 1

| Bsp. Nr. | A | $-Y-R^1$ | $R^2$ | $R^3$ | $R^8$ | $R^9$ | E | Fp. (°C) |
|---|---|---|---|---|---|---|---|---|
| 176 | $CH_2$ | $-SCH_3$ | $-CH_2CH_2CH_3$ | H | $OCHF_2$ | $OCHF_2$ | CH | |
| 177 | " | " | " | " | $CH_3$ | Cl | " | |
| 178 | " | " | " | " | $OCH_3$ | Br | " | |
| 179 | " | " | " | " | $OCH_3$ | $NHCH_3$ | " | |
| 180 | " | " | " | " | $OCHF_2$ | $OCH_3$ | " | |
| 181 | " | " | " | $CH_3$ | $OCH_3$ | $OCH_3$ | " | |
| 182 | " | " | " | " | $CH_3$ | $CH_3$ | " | |
| 183 | " | " | " | H | $OCH_3$ | $OCH_3$ | N | |
| 184 | " | " | " | " | $CH_3$ | $CH_3$ | N | |
| 185 | " | $-SC_6H_4-4-Cl$ | $-CH_3$ | " | $CH_3$ | $CH_3$ | CH | |
| 186 | " | " | " | " | $OCH_3$ | $CH_3$ | " | |
| 187 | " | " | " | " | $OCH_3$ | $OCH_3$ | " | 151-153 |
| 188 | " | " | " | " | " | Cl | " | |
| 189 | " | " | " | " | $OCHF_2$ | $CH_3$ | " | |
| 190 | " | " | " | " | $OCHF_2$ | $CF_3$ | " | |
| 191 | " | " | " | " | $OCHF_2$ | $OCHF_2$ | " | |
| 192 | " | " | " | " | $CH_3$ | Cl | " | |
| 193 | " | " | " | " | $OCH_3$ | Br | " | |
| 194 | " | " | " | " | $OCH_3$ | $NHCH_3$ | " | |
| 195 | " | " | " | " | $OCHF_2$ | $OCH_3$ | " | |
| 196 | " | | " | " | $CH_3$ | $CH_3$ | " | |
| 197 | " | " | " | " | $OCH_3$ | $CH_3$ | " | |
| 198 | " | " | " | " | $OCH_3$ | $OCH_3$ | " | |
| 199 | " | " | " | " | $OCH_3$ | Cl | " | |
| 200 | " | " | " | " | $OCHF_2$ | $CH_3$ | " | |
| 201 | " | " | " | " | $OCHF_2$ | $CF_3$ | " | |

Forts. Tabelle 1

| Bsp. Nr. | A | -Y-$R^1$ | $R^2$ | $R^3$ | $R^8$ | $R^9$ | E | Fp. (°C) |
|---|---|---|---|---|---|---|---|---|
| 202 | $CH_2$ | $-SCH_2C_6H_4$-2-Cl | $-CH_3$ | H | $OCHF_2$ | $OCHF_2$ | CH | |
| 203 | " | " | " | " | $CH_3$ | Cl | " | |
| 204 | " | " | " | " | $OCH_3$ | Br | " | |
| 205 | " | " | " | " | $OCH_3$ | $NHCH_3$ | " | |
| 206 | " | " | " | " | $OCHF_2$ | $OCH_3$ | " | |
| 207 | " | " | " | $CH_3$ | $OCH_3$ | $OCH_3$ | " | |
| 208 | " | " | " | " | $CH_3$ | $CH_3$ | " | |
| 209 | " | " | " | H | $OCH_3$ | $OCH_3$ | N | |
| 210 | " | " | " | " | $CH_3$ | $CH_3$ | N | |
| 211 | $CH_2CH_2$ | $-SO_2C_2H_5$ | $-CH_2CH_2CH_3$ | " | $CH_3$ | $CH_3$ | CH | 127-130 |
| 212 | " | " | " | " | $OCH_3$ | $CH_3$ | " | |
| 213 | " | " | " | " | $OCH_3$ | $OCH_3$ | " | |
| 214 | " | " | " | " | $OCH_3$ | Cl | " | |
| 215 | " | " | " | " | $OCHF_2$ | $CH_3$ | " | |
| 216 | " | " | " | " | $OCHF_2$ | $CF_3$ | " | |
| 217 | " | " | " | " | $OCHF_2$ | $OCHF_2$ | " | |
| 218 | " | " | " | " | $CH_3$ | Cl | " | |
| 219 | " | " | " | " | $OCH_3$ | Br | " | |
| 220 | " | " | " | " | $OCH_3$ | $NHCH_3$ | " | |
| 221 | " | " | " | " | $OCHF_2$ | $OCH_3$ | " | |
| 222 | " | " | $-CH(CH_3)_2$ | " | $CH_3$ | $CH_3$ | " | 108-111 |
| 223 | " | " | " | " | $OCH_3$ | $CH_3$ | " | |
| 224 | " | " | " | " | $OCH_3$ | $OCH_3$ | " | |
| 225 | " | " | " | " | $OCH_3$ | Cl | " | |
| 226 | " | " | " | " | $OCHF_2$ | $CH_3$ | " | |

Forts. Tabelle 1

| Bsp. Nr. | A | $-Y-R^1$ | $R^2$ | $R^3$ | $R^8$ | $R^9$ | E | Fp. (°C) |
|---|---|---|---|---|---|---|---|---|
| 227 | $CH_2CH_2$ | $-SO_2C_2H_5$ | $-CH(CH_3)_2$ | H | $OCHF_2$ | $CF_3$ | CH | |
| 228 | " | " | " | " | $OCHF_2$ | $OCHF_2$ | " | |
| 229 | " | " | " | " | $CH_3$ | Cl | " | |
| 230 | " | " | " | " | $OCH_3$ | Br | " | |
| 231 | " | " | " | " | $OCH_3$ | $NHCH_3$ | " | |
| 232 | " | " | " | " | $OCHF_2$ | $OCH_3$ | " | |
| 233 | " | " | $-CH_3$ | " | $CH_3$ | $CH_3$ | " | 144-146 |
| 234 | " | " | " | " | $OCH_3$ | $CH_3$ | " | |
| 235 | " | " | " | " | $OCH_3$ | $OCH_3$ | " | 150-151 |
| 236 | " | " | " | " | $OCH_3$ | Cl | " | |
| 237 | " | " | " | " | $OCHF_2$ | $CH_3$ | " | |
| 238 | " | " | " | " | " | $CF_3$ | " | |
| 239 | " | " | " | " | " | $OCHF_2$ | " | |
| 240 | " | " | " | " | $CH_3$ | Cl | " | |
| 241 | " | " | " | " | $OCH_3$ | Br | " | |
| 242 | " | " | " | " | $OCH_3$ | $NHCH_3$ | " | |
| 243 | " | " | " | " | $OCHF_2$ | $OCH_3$ | " | |
| 244 | " | " | $-(CH_2)_3CH_3$ | " | $CH_3$ | $CH_3$ | " | 106-109 |
| 245 | " | " | " | " | $OCH_3$ | $CH_3$ | " | |
| 246 | " | " | " | " | $OCH_3$ | $OCH_3$ | " | |
| 247 | " | " | " | " | $OCH_3$ | Cl | " | |
| 248 | " | " | " | " | $OCHF_2$ | $CH_3$ | " | |
| 249 | " | " | " | " | $OCHF_2$ | $CF_3$ | " | |
| 250 | " | " | " | " | $OCHF_2$ | $OCHF_2$ | " | |
| 251 | " | " | " | " | $CH_3$ | Cl | " | |
| 252 | " | " | " | " | $OCH_3$ | Br | " | |

Forts. Tabelle 1

| Bsp. Nr. | A | -Y-$R^1$ | $R^2$ | $R^3$ | $R^8$ | $R^9$ | E | Fp. (°C) |
|---|---|---|---|---|---|---|---|---|
| 253 | $CH_2CH_2$ | $-SO_2C_2H_5$ | $-(CH_2)_3CH_3$ | H | $OCH_3$ | $NHCH_3$ | CH | |
| 254 | " | " | " | " | $OCHF_2$ | $OCH_3$ | " | |
| 255 | " | $-SO_2C_6H_5$ | $-CH_2CO_2C_2H_5$ | " | $CH_3$ | $CH_3$ | " | Oel |
| 256 | " | " | " | " | $OCH_3$ | $CH_3$ | " | |
| 257 | " | " | " | " | $OCH_3$ | $OCH_3$ | " | |
| 258 | " | " | " | " | $OCH_3$ | Cl | " | |
| 259 | " | " | " | " | $OCHF_2$ | $CH_3$ | " | |
| 260 | " | " | " | " | $OCHF_2$ | $CF_3$ | " | |
| 261 | " | " | " | " | " | $OCHF_2$ | " | |
| 262 | " | " | " | " | $CH_3$ | Cl | " | |
| 263 | " | " | " | " | $OCH_3$ | Br | " | |
| 264 | " | " | " | " | " | $NHCH_3$ | " | |
| 265 | " | " | " | " | $OCHF_2$ | $OCH_3$ | " | |
| 266 | " | $-SO_2C_2H_5$ | $-C_6H_{11}$ | " | $CH_3$ | $CH_3$ | " | 156-158 |
| 267 | " | " | " | " | $OCH_3$ | $CH_3$ | " | |
| 268 | " | " | " | " | $OCH_3$ | $OCH_3$ | " | |
| 269 | " | " | " | " | $OCH_3$ | Cl | " | |
| 270 | " | " | " | " | $OCHF_2$ | $CH_3$ | " | |
| 271 | " | " | " | " | $OCHF_2$ | $CF_3$ | " | |
| 272 | " | " | " | " | $OCHF_2$ | $OCHF_2$ | " | |
| 273 | " | " | " | " | $CH_3$ | Cl | " | |
| 274 | " | " | " | " | $OCH_3$ | Br | " | |
| 275 | " | " | " | " | $OCH_3$ | $NHCH_3$ | " | |
| 276 | " | " | " | " | $OCHF_2$ | $OCH_3$ | " | |

Forts. Tabelle 1

| Bsp. Nr. | A | -Y-R[1] | $R^2$ | $R^3$ | $R^8$ | $R^9$ | E | Fp. (°C) |
|---|---|---|---|---|---|---|---|---|
| 277 | $CH_2CH_2$ | $-SO_2CH_3$ | $-CH_3$ | H | $CH_3$ | $CH_3$ | CH | |
| 278 | " | " | " | " | $OCH_3$ | $CH_3$ | " | |
| 279 | " | " | " | " | " | $OCH_3$ | " | |
| 280 | " | " | " | " | " | Cl | " | |
| 281 | " | " | " | " | $OCHF_2$ | $CH_3$ | " | |
| 282 | " | " | " | " | $OCHF_2$ | $CF_3$ | " | |
| 283 | " | " | " | " | $OCHF_2$ | $OCHF_2$ | " | |
| 284 | " | " | " | " | $CH_3$ | Cl | " | |
| 285 | " | " | " | " | $OCH_3$ | Br | " | |
| 286 | " | " | " | " | $OCH_3$ | $NHCH_3$ | " | |
| 287 | " | " | " | " | $OCHF_2$ | $OCH_3$ | " | |
| 288 | " | " | $-CH_2CH_3$ | " | $CH_3$ | $CH_3$ | " | |
| 289 | " | " | " | " | $OCH_3$ | $CH_3$ | " | |
| 290 | " | " | " | " | " | OCH3 | " | |
| 291 | " | " | " | " | " | Cl | " | |
| 292 | " | " | " | " | $OCHF_2$ | $CH_3$ | " | |
| 293 | " | " | " | " | " | $CF_3$ | " | |
| 294 | " | " | " | " | " | $OCHF_2$ | " | |
| 295 | " | " | " | " | $CH_3$ | Cl | " | |
| 296 | " | " | " | " | $OCH_3$ | Br | " | |
| 297 | " | " | " | " | $OCH_3$ | $NHCH_3$ | " | |
| 298 | " | " | " | " | $OCHF_2$ | $OCH_3$ | " | |
| 299 | $CH_2$ | $-SCH_3$ | $-CH_3$ | H | $OC_2H_5$ | $OC_2H_5$ | " | 128 |
| 300 | " | $-SO_2CH_3$ | " | " | " | " | " | |
| 301 | " | $-SC_2H_5$ | " | " | " | " | " | 119 |

Forts. Tabelle 1

| Bsp. Nr. | A | $-Y-R^1$ | $R^2$ | $R^3$ | $R^8$ | $R^9$ | E | Fp. (°C) |
|---|---|---|---|---|---|---|---|---|
| 302 | $CH_2$ | $-SO_2C_2H_5$ | $-CH_3$ | H | $OC_2H_5$ | $OC_2H_5$ | CH | |
| 303 | " | $-SCH_2C_6H_5$ | " | " | " | " | " | |
| 304 | " | $-SO_2CH_2C_6H_5$ | " | " | " | " | " | |
| 305 | " | $-SCH_2CO_2CH_3$ | " | " | " | " | " | |
| 306 | " | $-SCOCH_3$ | " | " | " | " | " | |
| 307 | " | $-SCH_2CH_2CO_2CH_3$ | " | " | " | " | " | |
| 308 | " | $-SC_6H_4-2-CO_2C_2H_5$ | " | " | " | " | " | |
| 309 | " | $-SC_6H_4-2-Cl$ | " | " | " | " | " | |
| 310 | " | $-SCH_2C_6H_4-2-Cl$ | " | " | " | " | " | |
| 311 | " | " | $-C_2H_5$ | " | " | " | " | |
| 312 | " | $-SC_6H_4-2-Cl$ | " | " | " | " | " | |
| 313 | " | $-SC_6H_4-2-CO_2CH_3$ | " | " | " | " | " | |
| 314 | " | $-SCH_2CH_2CO_2CH_3$ | " | " | " | " | " | |
| 315 | " | $-SCH_2CO_2CH_3$ | " | " | " | " | " | |
| 316 | " | $-SO_2CH_2C_6H_5$ | " | " | " | " | " | |
| 317 | " | $-SCH_2C_6H_5$ | " | " | " | " | " | |
| 318 | " | $-SO_2C_2H_5$ | " | " | " | " | " | |
| 319 | " | $-SC_2H_5$ | " | " | " | " | " | |
| 320 | " | $-SO_2CH_3$ | " | " | " | " | " | |
| 321 | " | $-SCH_3$ | " | " | " | " | " | |

**Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0 bis 5 ausgedrückt ist. Dabei bedeutet:

0 = ohne Wirkung
1 = 0 bis 20 % Wirkung bzw. Schaden
2 = 20 bis 40 % Wirkung bzw. Schaden
3 = 40 bis 60 % Wirkung bzw. Schaden
4 = 60 bis 80 % Wirkung bzw. Schaden
5 = 80 bis 100 % Wirkung bzw. Schaden

### 1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspension bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Boniturwerte in Tabelle I zeigen, weisen die erfindungsgemäßen Verbindungen eine gute herbizide Vorauflaufwirksamkeit gegen ein breites Spektrum von Ungräsern und Unkräutern auf.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle II).

3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die Verbindungen der Formel I weisen somit eine hohe Selektivität bei Anwendung zur Bekämpfung von unerwünschten Pflanzenwuchs in landwirtschaftlichen Kulturen auf.

Tabelle I

| Vorauflaufwirkung der erfindungsgemäßen Verbindungen | | | | | |
|---|---|---|---|---|---|
| Beispiel-Nr. | Dosis (kg a.i./ha) | herbizide Wirkung | | | |
| | | STM | CRS | SIA | LOM |
| 92 | 0,6 | 2 | 4 | 4 | 2 |
| 106 | 0,6 | 3 | 4 | 5 | 2 |
| 81 | 0,6 | 3 | 5 | 4 | 4 |
| 117 | 0,6 | 2 | 5 | 2 | 2 |
| 25 | 0,6 | 3 | 5 | 3 | 3 |
| 172 | 0,6 | 5 | 3 | 5 | 3 |
| 170 | 0,6 | 5 | 5 | 5 | 5 |
| 3 | 0,6 | 5 | 5 | 5 | 5 |
| 14 | 0,6 | 3 | 5 | 4 | 4 |
| 235 | 0,6 | 5 | 5 | 5 | 4 |
| 211 | 0,6 | 5 | 5 | 5 | 3 |
| 244 | 0,6 | 2 | 3 | 5 | 2 |
| 222 | 0,6 | 1 | 4 | 4 | 2 |
| 233 | 0,6 | 5 | 5 | 5 | 3 |
| 266 | 0,6 | 2 | 5 | 5 | 4 |

STM = Stellaria media
CRS = Chrysanthemum segetum
SIA = Sinapis alba
LOM = Lolium multiflorum

Tabelle II

| Nachauflaufwirkung der erfindungsgemäßen Verbindungen | | | | | |
|---|---|---|---|---|---|
| Beispiel-Nr. | Dosis (kg a.i./ha) | herbizide Wirkung | | | |
| | | STM | CRS | SIA | LOM |
| 92 | 0,6 | 2 | 3 | 5 | 1 |
| 1 | 0,6 | 3 | 3 | 4 | 2 |
| 106 | 0,6 | 4 | 4 | 5 | 1 |
| 108 | 0,6 | 3 | 2 | 4 | 2 |
| 81 | 0,6 | 4 | 4 | 4 | 2 |
| 119 | 0,6 | 5 | 2 | 2 | 1 |
| 25 | 0,6 | 4 | 2 | 4 | 2 |
| 172 | 0,6 | 3 | 3 | 3 | 2 |
| 170 | 0,6 | 5 | 5 | 4 | 4 |
| 3 | 0,6 | 5 | 4 | 4 | 3 |
| 14 | 0,6 | 5 | 5 | 5 | 2 |
| 235 | 0,6 | 4 | 5 | 5 | 4 |
| 211 | 0,6 | 5 | 4 | 4 | 3 |

**Wuchshemmung an Getreide**

In Schalenversuchen im Gewächshaus wurden junge Getreidepflanzen (Weizen, Gerste, Roggen) im 3-Blattstadium mit erfindungsgemäßen Verbindungen in verschiedenen Wirkstoffkonzentrationen (kg/ha) tropfnass gespritzt.

Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet, wobei 100 % den Stillstand des Wachstums und 0 % ein Wachstum entsprechend den unbehandelten Kontrollpflanzen bedeuten. Es zeigte sich, daß die Verbindungen sehr gute wachstumsregulierende Eigenschaften besitzen.

**Patentansprüche**

**Patentansprüche für folgende Vertragsstaaten : AT, BE, CH, DE, FR, GB, GR, IT, LI, NL**

**1.** Verbindung der allgemeinen Formel (I)

$$R^1-Y-A-NR^2-SO_2-NH-\overset{Z}{\overset{\|}{C}}-NR^3R^4 \qquad (I)$$

worin

| | |
|---|---|
| A | einen gesättigten oder ungesättigten, unverzweigten oder verzweigten $C_1$-$C_{10}$-Kohlenwasserstoffrest, |
| $R^1$ | $C_1$-$C_8$-Alkyl, $C_2$-$C_8$-Alkenyl, $C_2$-$C_8$-Alkinyl, $C_3$-$C_8$-Cycloalkyl, $C_5$-$C_8$-Cycloalkenyl oder einen der vorstehenden fünf Reste, der ein- oder mehrfach durch Halogen oder durch solche Reste substituiert ist, die aus der Gruppe aus $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfinyl, $C_1$-$C_6$-Alkylsulfonyl, $C_3$-$C_6$-Cycloalkyl, einem Rest eines drei- bis sechsgliedrigen gesättigten Heterozyklus mit einem Sauerstoffatom im Ring, Furyl, Phenyl und einem Phenylrest, der ein- oder mehrfach durch Reste aus der Gruppe aus Halogen, $C_1$-$C_4$-Alkyl, $C_1$-$C_4$-Alkoxy, $CF_3$, ($C_1$-$C_4$-Alkoxy)-carbonyl und Nitro substituiert ist, ausgewählt sind, oder |
| $R^1$ | Phenyl oder einen Phenylrest, der ein- oder mehrfach durch Reste aus der Gruppe bestehend aus Halogen, $C_1$-$C_4$-Alkyl, $C_1$-$C_4$-Alkoxy, $CF_3$, ($C_1$-$C_4$-Alkoxy)-carbonyl und Nitro substituiert ist, oder |
| $R^1$ | einen Rest der Formel<br><br>$-CO-R^5$, $-CHR^6-COOR^7$, $-CHR^6-CH_2-COOR^7$ oder $-CH_2-CHR^6-COOR^7$,<br><br>wobei in den Formeln $R^5$ für $C_1$-$C_6$-Alkyl, $R^6$ für Wasserstoff, $C_1$-$C_4$-Alkyl, Phenyl oder Benzyl und $R^7$ für H, $C_1$-$C_4$-Alkyl, Phenyl oder Benzyl stehen, |
| Y | S oder $SO_2$, |
| $R^2$ | H, $C_1$-$C_8$-Alkyl, $C_2$-$C_8$-Alkenyl, $C_2$-$C_8$-Alkinyl oder einen der vorstehenden drei Reste, der ein- oder mehrfach durch Halogen oder durch Reste aus der Gruppe aus $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfinyl, $C_1$-$C_6$-Alkylsulfonyl, ($C_1$-$C_6$-Alkoxy)-carbonyl, Phenoxycarbonyl, Benzyloxycarbonyl und Phenyl substituiert ist,<br>$C_3$-$C_8$-Cycloalkyl, das unsubstituiert ist oder ein- oder mehrfach durch Halogen oder ein- oder zweifach durch $C_1$-$C_4$-Alkoxy oder $C_1$-$C_4$-Alkylthio substituiert ist, $C_5$-$C_8$-Cycloalkenyl, Cyclopropylmethyl, Epoxypropyl, Furfuryl, Tetrahydrofurfuryl, Phenoxy-$C_1$-$C_6$-alkyl, Phenyl oder einen der letzten zwei vorstehenden Reste, der im Phenylring durch Halogen, $C_1$-$C_4$-Alkyl, $C_1$-$C_4$-Alkoxy oder Nitro substituiert ist, |
| $R^3$ | H, $C_1$-$C_8$-Alkyl, $C_2$-$C_8$-Alkenyl, $C_2$-$C_8$-Alkinyl oder $C_1$-$C_4$-Alkoxy, |
| $R^4$ | einen Rest der Formel |

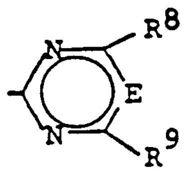 , 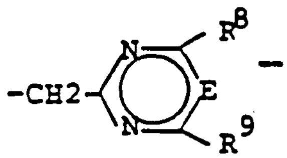 , 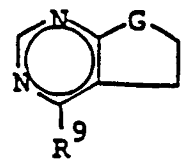 ,

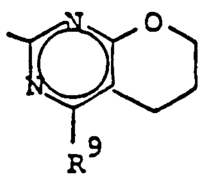 , 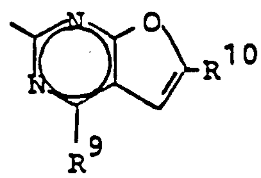 , 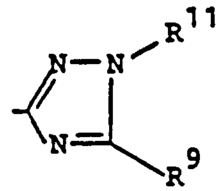 ,

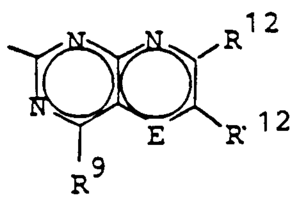 oder 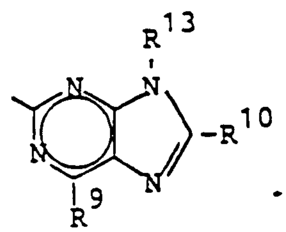 .

| | |
|---|---|
| $R^8$ und $R^9$ | unabhängig voneinander H, Halogen, $C_1$-$C_6$-Alkyl, $C_1$-$C_6$-Alkoxy, $C_1$-$C_6$-Alkylthio oder einen der letzten drei vorstehenden Reste, der ein- oder mehrfach durch Halogen oder ein- oder zweifach durch $C_1$-$C_4$-Alkoxy oder $C_1$-$C_4$-Alkylthio substituiert ist, oder einen Rest $NR^{14}R^{15}$, $C_3$-$C_6$-Cycloalkyl, -$OCHR^{16}COOR^{17}$, $C_3$-$C_5$-Alkenyl, $C_2$-$C_4$-Alkinyl, $C_3$-$C_5$-Alkenyloxy oder $C_3$-$C_5$-Alkinyloxy, |
| $R^{10}$ | Wasserstoff oder $C_1$-$C_4$-Alkyl, |
| $R^{11}$ | $C_1$-$C_4$-Alkyl, -$CHF_2$ oder -$CH_2CF_3$, |
| $R^{12}$ | unabhängig voneinander H, $C_1$-$C_4$-Alkyl, $C_1$-$C_4$-Alkoxy oder Halogen, |
| $R^{13}$ | Wasserstoff, $C_1$-$C_4$-Alkyl, $CHF_2$ oder $CH_2CF_3$, |
| $R^{14}$ und $R^{15}$ | unabhängig voneinander H, $C_1$-$C_4$-Alkyl, $C_2$-$C_4$-Alkenyl oder $C_3$-$C_4$-Alkinyl, |
| $R^{16}$ | Wasserstoff oder $C_1$-$C_4$-Alkyl, |
| $R^{17}$ | Wasserstoff oder $C_1$-$C_4$-Alkyl, |
| E | CH oder N, |
| G | $CH_2$ oder O und |
| Z | O oder S bedeuten, sowie ihre Salze. |

**2.** Verbindung nach Anspruch 1, dadurch gekennzeichnet, daß

$R^1$ $C_1$-$C_4$-Alkyl, einen $C_1$-$C_4$-Alkylrest, der ein- oder mehrfach durch Halogen oder ein- oder zweifach durch $C_1$-$C_4$-Alkoxy, $C_2$-$C_3$-Alkenyloxy, $C_2$-$C_3$-Alkinyloxy, $C_1$-$C_4$-Alkylthio, $C_1$-$C_4$-Alkylsulfinyl, $C_1$-$C_4$-Alkylsulfonyl, Phenyl oder einen Phenylrest, der ein-bis dreifach durch Reste aus der Gruppe aus Halogen, $C_1$-$C_4$-Alkyl, $C_1$-$C_4$-Alkoxy, $CF_3$, ($C_1$-$C_4$-Alkoxy)-carbonyl und Nitro subtituiert ist, substituiert ist, oder $C_3$-$C_8$-Cycloalkyl oder ein $C_3$-$C_8$-Cycloalkylrest, der ein- oder mehrfach durch Halogen oder ein- bis zweifach durch $C_1$-$C_4$-Alkoxy oder $C_1$-$C_4$-Alkylthio substituiert ist, oder

$C_5$-$C_8$-Cycloalkenyl, Cyclopropylmethyl, Epoxypropyl, Furfuryl, Tetrahydrofurfuryl, Benzyl, Phenyl, oder einen Benzyl- oder Phenylrest, der im Phenylring durch einen oder mehrere Reste aus der Gruppe aus Halogen, $C_1$-$C_4$-Alkyl, $C_1$-$C_4$-Alkoxy, $CF_3$, ($C_1$-$C_4$-Alkoxy)-carbonyl und Nitro substituiert ist, oder

einen Rest der Formel

-$COR^5$, $CHR^6$-$COOR^7$, $CHR^6$-$CH_2$-$COOR^7$ oder $CH_2$-$CHR^6$-$COOR^7$,

$R^5$ $C_1$-$C_4$-Alkyl,

$R^6$ H, $C_1$-$C_4$-Alkyl, Benzyl oder Phenyl und

$R^7$ H, $C_1$-$C_4$-Alkyl, Benzyl oder Phenyl bedeuten.

**3.** Verbindung nach Anspruch 1 oder 2, dadurch gekennzeichnet, daß

$R^2$ $C_1$-$C_4$-Alkyl, einen $C_1$-$C_4$-Alkylrest, der ein- oder mehrfach durch Halogen oder ein- oder zweifach durch $C_1$-$C_4$-Alkoxy, $C_2$-$C_4$-Alkenyloxy, Propargyloxy, $C_1$-$C_4$-Alkylthio, $C_1$-$C_4$-Alkylsulfinyl, $C_1$-$C_4$-Alkylsulfonyl, ($C_1$-$C_4$-Alkoxy)-carbonyl, Phenoxycarbonyl, Benzyloxycarbonyl oder Phenyl substituiert ist, oder $C_3$-$C_8$-Cycloalkyl bedeutet.

**4.** Verbindung nach einem oder mehreren der Ansprüche 1 bis 3, dadurch gekennzeichnet, daß

$R^4$ einen Rest der Formel

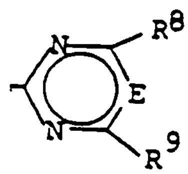

,

und

$R^8$ und $R^9$ unabhängig voneinander Halogen, $C_1$-$C_4$-Alkyl, $C_1$-$C_4$-Alkoxy, $C_1$-$C_4$-Alkylthio oder einen der letzten drei vorstehenden Reste, die ein- oder mehrfach durch Halogen oder ein- oder zweifach durch $C_1$-$C_4$-Alkoxy, $C_1$-$C_4$-Alkylthio substituiert sind, oder einen Rest $NR^{14}R^{15}$, $C_3$-$C_6$-Cycloalkyl, -$OCHR^{16}$ $COOR^{17}$, Allyl, Propargyl, Allyloxy oder Propargyloxy bedeuten,

$R^{14}$ und $R^{15}$ unabhängig voneinander H oder $C_1$-$C_4$-Alkyl,

$R^{16}$ H oder $C_1$-$C_4$-Alkyl,

$R^{17}$ $C_1$-$C_4$-Alkyl und

E CH oder N bedeuten.

**5.** Verbindung nach einem oder mehreren der Ansprüche 1 bis 4, dadurch gekennzeichnet, daß

A ein Rest der Formel $CH_2$, $CH_2CH_2$, CHR, CRR', $CH_2CHR$ oder $CH_2CRR'$ ist, wobei R und R' unabhängig voneinander $C_1$-$C_4$-Alkyl oder $C_2$-$C_4$-Alkenyl bedeuten.

**6.** Verbindung nach einem oder mehreren der Ansprüche 1 bis 5, dadurch gekennzeichnet, daß

A einen Rest der Formel -$CH_2$- oder -$CH_2$-$CH_2$-,

$R^1$ $C_1$-$C_4$-Alkyl, einen $C_1$-$C_4$-Alkylrest, der ein- oder mehrfach durch Halogen oder ein- bis zweifach durch $C_1$-$C_4$-Alkoxy substituiert ist,

$C_3$-$C_8$-Cycloalkyl, das ein- oder mehrfach durch Halogen substituiert ist oder unsubstituiert ist,

Benzyl, Phenyl oder einen Benzyl- oder Phenylrest, der im Phenylring ein- oder mehrfach durch Halogen, $C_1$-$C_4$-Alkyl, $C_1$-$C_4$-Alkoxy, $CF_3$, ($C_1$-$C_4$-Alkoxy)-carbonyl oder Nitro substituiert ist oder

einen Rest der Formel

-$CR^6H$-$CO_2R^7$

worin $R^6$ und $R^7$ gleich oder verschieden sind und jeweils H, $C_1$-$C_4$-Alkyl, Phenyl oder Benzyl bedeuten,

$R^2$ $C_1$-$C_4$-Alkyl, das unsubstituiert ist oder ein- oder mehrfach durch Halogen oder durch ($C_1$-$C_4$-Alkoxyl)-carbonyl, Phenyloxycarbonyl, Benzyloxycarbonyl oder Phenyl substituiert ist,

$R^3$ H, $C_1$-$C_4$-Alkyl oder Allyl, insbesondere H,

$R^4$ einen Rest der Formel

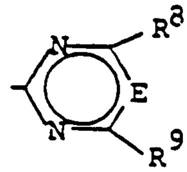 ,

$R^8$ und $R^9$ unabhängig voneinander Halogen, $C_1$-$C_4$-Alkyl, $C_1$-$C_4$-Alkoxy oder einen der letzten beiden vorstehenden Reste, der halogeniert ist,

E CH oder N und

Z O oder S bedeuten.

7. Herbizides Mittel, dadurch gekennzeichnet, daß es eine Verbindung der Formel (I) oder eines ihrer Salze nach einem oder mehreren der Ansprüche 1 bis 6 neben inerten Trägerstoffen enthält.

8. Pflanzenwachstumsregulierendes Mittel, dadurch gekennzeichnet, daß es eine Verbindung der Formel (I) oder eines ihrer Salze nach mindestens einem der Ansprüche 1 bis 6 neben inerten Trägerstoffen enthält.

9. Verwendung von Verbindungen der Formel (I) und eines ihrer Salze gemäß einem oder mehreren der Ansprüche 1 bis 6 als Herbizide oder Pflanzenwachstumsregulatoren.

10. Verfahren zur Herstellung einer Verbindung der Formel (I) nach Anspruch 1, dadurch gekennzeichnet, daß man
(a) eine Verbindung der Formel (II)

$R^1$-Y-A-$NR^2$-$SO_2$-N = C = Z (II),

mit einer Verbindung der Formel (III)

H-$NR^3R^4$ (III),

umsetzt, wobei in den Formeln (II) und (III) A, Y, Z, $R^1$, $R^2$, $R^3$ und $R^4$ die bei Formel (I) angegebenen Bedeutungen haben, oder
(b) eine Verbindung der Formel (IV)

$R^1$-Y-A-$NR^2$-$SO_2$-$NH_2$ (IV)

mit einem Carbamat bzw. Thiocarbamat der Formel (V)

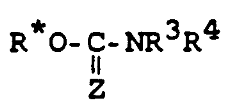 (V)

umsetzt,
wobei in den Formeln (IV) und (V) $R^1$, $R^2$, $R^3$, $R^4$, A, Y und Z die bei Formel (I) angegebenen Bedeutungen haben und $R^*$ $C_1$-$C_6$-Alkyl, $C_1$-$C_4$-Halogenalkyl, Phenyl oder einen Phenylrest bedeutet, der ein- oder mehrfach durch Halogen, $C_1$-$C_4$-Alkyl oder Nitro subtituiert ist, oder
(c) ein Carbamat bzw. Thiocarbamat der Formel (VI) mit einer Verbindung der oben genannten Formel (III)

$$R^1\text{-}Y\text{-}A\text{-}NR^2\text{-}SO_2\text{-}NH\text{-}\underset{\underset{Z}{\|}}{C}\text{-}OR^* \qquad (VI)$$

umsetzt, wobei $R^1$, $R^2$, $R^*$, Y, A und Z die genannten Bedeutungen haben, oder
(d) eine Verbindung der Formel (VII) oder (VIII)

$R^1$-Y-A-NH-$R^2$ (VII)

$R^1$-Y-A-NH-$R^2$ x HCl (VIII)

mit einer Verbindung der Formel (IX)

$$ClSO_2\text{-}NH\text{-}\underset{\underset{Z}{\|}}{C}\text{-}NR^3R^4 \qquad (IX)$$

umsetzt, wobei in den Formeln (VII) bis (IX) A, Y, Z, $R^1$, $R^2$, $R^3$ und $R^4$ die oben angegebenen Bedeutungen haben.

**Patentansprüche für folgenden Vertragsstaat : ES**

**1.** Verwendung der Verbindung der allgemeinen Formel (I)

$$R^1\text{-}Y\text{-}A\text{-}NR^2\text{-}SO_2\text{-}NH\text{-}\overset{\overset{Z}{\|}}{C}\text{-}NR^3R^4 \qquad (I)$$

worin

A einen gesättigten oder ungesättigten, unverzweigten oder verzweigten $C_1$-$C_{10}$-Kohlenwasserstoffrest,

$R^1$ $C_1$-$C_8$-Alkyl, $C_2$-$C_8$-Alkenyl, $C_2$-$C_8$-Alkinyl, $C_3$-$C_8$-Cycloalkyl, $C_5$-$C_8$-Cycloalkenyl oder einen der vorstehenden fünf Reste, der ein- oder mehrfach durch Halogen oder durch solche Reste substituiert ist, die aus der Gruppe aus $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfinyl, $C_1$-$C_6$-Alkylsulfonyl, $C_3$-$C_6$-Cycloalkyl, einem Rest eines drei- bis sechsgliedrigen gesättigten Heterozyklus mit einem Sauerstoffatom im Ring, Furyl, Phenyl und einem Phenylrest, der ein- oder mehrfach durch Reste aus de Gruppe aus Halogen, $C_1$-$C_4$-Alkyl, $C_1$-$C_4$-Alkoxy, $CF_3$, ($C_1$-$C_4$-Alkoxy)-carbonyl und Nitro substituiert ist, ausgewählt sind, oder

$R^1$ Phenyl oder einen Phenylrest, der ein- oder mehrfach durch Reste aus der Gruppe bestehend aus Halogen, $C_1$-$C_4$-Alkyl, $C_1$-$C_4$-Alkoxy, $CF_3$, ($C_1$-$C_4$-Alkoxy)-carbonyl und Nitro substituiert ist, oder

$R^1$ einen Rest der Formel

-CO-$R^5$, -$CHR^6$-$COOR^7$, -$CHR^6$-$CH_2$-$COOR^7$ oder -$CH_2$-$CHR^6$-$COOR^7$,

wobei in den Formeln $R^5$ für $C_1$-$C_6$-Alkyl, $R^6$ für Wasserstoff, $C_1$-$C_4$-Alkyl, Phenyl oder Benzyl und $R^7$ für H, $C_1$-$C_4$-Alkyl, Phenyl oder Benzyl stehen,

Y S oder $SO_2$,

$R^2$ H, $C_1$-$C_8$-Alkyl, $C_2$-$C_8$-Alkenyl, $C_2$-$C_8$-Alkinyl oder einen der vorstehenden drei Reste, der ein- oder mehrfach durch Halogen oder durch Reste aus der Gruppe aus $C_1$-$C_6$-Alkoxy, $C_2$-$C_6$-Alkenyloxy, $C_2$-$C_6$-Alkinyloxy, $C_1$-$C_6$-Alkylthio, $C_1$-$C_6$-Alkylsulfi-

nyl, $C_1$-$C_6$-Alkylsulfonyl, ($C_1$-$C_6$-Alkoxy)-carbonyl, Phenoxycarbonyl, Benzyloxycarbonyl und Phenyl substituiert ist,
$C_3$-$C_8$-Cycloalkyl, das unsubstituiert ist oder ein- oder mehrfach durch Halogen oder ein- oder zweifach durch $C_1$-$C_4$-Alkoxy oder $C_1$-$C_4$-Alkylthio substituiert ist, $C_5$-$C_8$-Cycloalkenyl, Cyclopropylmethyl, Epoxypropyl, Furfuryl, Tetrahydrofurfuryl, Phenoxy-$C_1$-$C_6$-alkyl, Phenyl oder einen der letzten zwei vorstehenden Reste, der im Phenylring durch Halogen, $C_1$-$C_4$-Alkyl, $C_1$-$C_4$-Alkoxy oder Nitro substituiert ist,

$R^3$ H, $C_1$-$C_8$-Alkyl, $C_2$-$C_8$-Alkenyl, $C_2$-$C_8$-Alkinyl oder $C_1$-$C_4$-Alkoxy,

$R^4$ einen Rest der Formel

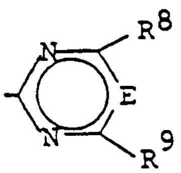 , 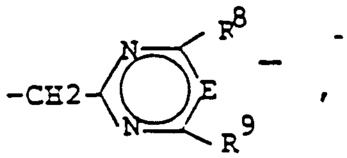 , 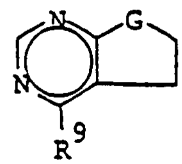 ,

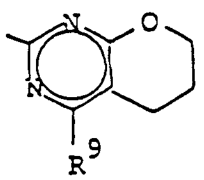 , 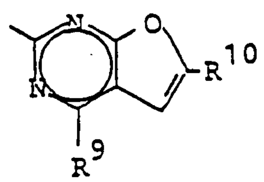 , 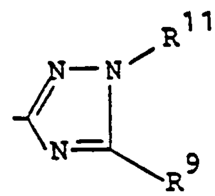 ,

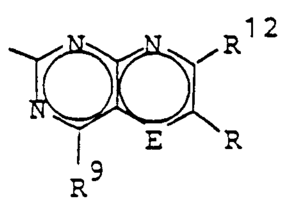 oder 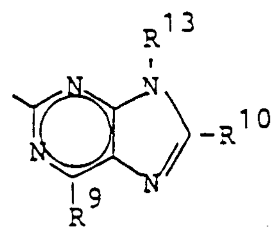

$R^8$ und $R^9$ unabhängig voneinander H, Halogen, $C_1$-$C_6$-Alkyl, $C_1$-$C_6$-Alkoxy, $C_1$-$C_6$-Alkylthio oder einen der letzten drei vorstehenden Reste, der ein- oder mehrfach durch Halogen oder ein- oder zweifach durch $C_1$-$C_4$-Alkoxy oder $C_1$-$C_4$-Alkylthio substituiert ist, oder einen Rest $NR^{14}R^{15}$, $C_3$-$C_6$-Cycloalkyl, $-OCHR^{16}COOR^{17}$, $C_3$-$C_5$-Alkenyl, $C_2$-$C_4$-Alkinyl, $C_3$-$C_5$-Alkenyloxy oder $C_3$-$C_5$-Alkinyloxy,

$R^{10}$ Wasserstoff oder $C_1$-$C_4$-Alkyl,

$R^{11}$ $C_1$-$C_4$-Alkyl, $-CHF_2$ oder $-CH_2CF_3$,

$R^{12}$ unabhängig voneinander H, $C_1$-$C_4$-Alkyl, $C_1$-$C_4$-Alkoxy oder Halogen,

$R^{13}$ Wasserstoff, $C_1$-$C_4$-Alkyl, $CHF_2$ oder $CH_2CF_3$,

$R^{14}$ und $R^{15}$ unabhängig voneinander H, $C_1$-$C_4$-Alkyl, $C_2$-$C_4$-Alkenyl oder $C_3$-$C_4$-Alkinyl,

$R^{16}$ Wasserstoff oder $C_1$-$C_4$-Alkyl,

$R^{17}$ Wasserstoff oder $C_1$-$C_4$-Alkyl,

E CH oder N,

G $CH_2$ oder O und

Z O oder S bedeuten, sowie ihrer Salze als Herbizide oder Pflanzenwachstumsregulatoren.

**2.** Verwendung nach Anspruch 1, dadurch gekennzeichnet, daß

$R^1$ $C_1$-$C_4$-Alkyl, einen $C_1$-$C_4$-Alkylrest, der ein- oder mehrfach durch Halogen oder ein- oder

zweifach durch $C_1$-$C_4$-Alkoxy, $C_2$-$C_3$-Alkenyloxy, $C_2$-$C_3$-Alkinyloxy, $C_1$-$C_4$-Alkylthio, $C_1$-$C_4$-Alkylsulfinyl, $C_1$-$C_4$-Alkylsulfonyl, Phenyl oder einen Phenylrest, der ein- bis dreifach durch Reste aus der Gruppe aus Halogen, $C_1$-$C_4$-Alkyl, $C_1$-$C_4$-Alkoxy, $CF_3$, ($C_1$-$C_4$-Alkoxy)-carbonyl und Nitro subtituiert ist, substituiert ist, oder $C_3$-$C_8$-Cycloalkyl oder ein $C_3$-$C_8$-Cycloalkylrest, der ein- oder mehrfach durch Halogen oder ein- bis zweifach durch $C_1$-$C_4$-Alkoxy oder $C_1$-$C_4$-Alkylthio substituiert ist, oder

$C_5$-$C_8$-Cycloalkenyl, Cyclopropylmethyl, Epoxypropyl, Furfuryl, Tetrahydrofurfuryl, Benzyl, Phenyl, oder einen Benzyl- oder Phenylrest, der im Phenylring durch einen oder mehrere Reste aus der Gruppe aus Halogen, $C_1$-$C_4$-Alkyl, $C_1$-$C_4$-Alkoxy, $CF_3$, ($C_1$-$C_4$-Alkoxy)-carbonyl und Nitro substituiert ist, oder

einen Rest der Formel

$-COR^5$, $CHR^6$-$COOR^7$, $CHR^6$-$CH_2$-$COOR^7$ oder $CH_2$-$CHR^6$-$COOR^7$,

$R^5$ $C_1$-$C_4$-Alkyl,

$R^6$ H, $C_1$-$C_4$-Alkyl, Benzyl oder Phenyl und

$R^7$ H, $C_1$-$C_4$-Alkyl, Benzyl oder Phenyl bedeuten.

**3.** Verwendung nach Anspruch 1 oder 2, dadurch gekennzeichnet, daß

$R^2$ $C_1$-$C_4$-Alkyl, einen $C_1$-$C_4$-Alkylrest, der ein- oder mehrfach durch Halogen oder ein- oder zweifach durch $C_1$-$C_4$-Alkoxy, $C_2$-$C_4$-Alkenyloxy, Propargyloxy, $C_1$-$C_4$-Alkylthio, $C_1$-$C_4$-Alkylsulfinyl, $C_1$-$C_4$-Alkylsulfonyl, ($C_1$-$C_4$-Alkoxy)-carbonyl, Phenoxycarbonyl, Benzyloxycarbonyl oder Phenyl substituiert ist, oder $C_3$-$C_8$-Cycloalkyl bedeutet.

**4.** Verwendung nach einem oder mehreren der Ansprüche 1 bis 3, dadurch gekennzeichnet, daß

$R^4$ einen Rest der Formel

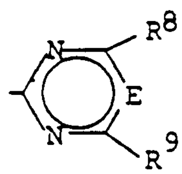 ,

und

$R^8$ und $R^9$ unabhängig voneinander Halogen, $C_1$-$C_4$-Alkyl, $C_1$-$C_4$-Alkoxy, $C_1$-$C_4$-Alkylthio oder einen der letzten drei vorstehenden Reste, die ein- oder mehrfach durch Halogen oder ein- oder zweifach durch $C_1$-$C_4$-Alkoxy, $C_1$-$C_4$-Alkylthio substituiert sind, oder einen Rest $NR^{14}R^{15}$, $C_3$-$C_6$-Cycloalkyl, $-OCHR^{16}$ $COOR^{17}$, Allyl, Propargyl, Allyloxy oder Propargyloxy bedeuten,

$R^{14}$ und $R^{15}$ unabhängig voneinander H oder $C_1$-$C_4$-Alkyl,

$R^{16}$ H oder $C_1$-$C_4$-Alkyl,

$R^{17}$ $C_1$-$C_4$-Alkyl und

E CH oder N bedeuten.

**5.** Verwendung nach einem oder mehreren der Ansprüche 1 bis 4, dadurch gekennzeichnet, daß

A ein Rest der Formel $CH_2$, $CH_2CH_2$, CHR, CRR′, $CH_2CHR$ oder $CH_2CRR′$ ist, wobei R und R′ unabhängig voneinander $C_1$-$C_4$-Alkyl oder $C_2$-$C_4$-Alkenyl bedeuten.

**6.** Verbindung nach einem oder mehreren der Ansprüche 1 bis 5, dadurch gekennzeichnet, daß

A einen Rest der Formel $-CH_2-$ oder $-CH_2-CH_2-$,

$R^1$ $C_1$-$C_4$-Alkyl, einen $C_1$-$C_4$-Alkylrest, der ein- oder mehrfach durch Halogen oder ein- bis zweifach durch $C_1$-$C_4$-Alkoxy substituiert ist,

$C_3$-$C_8$-Cycloalkyl, das ein- oder mehrfach durch Halogen substituiert ist oder unsubstituiert ist,

Benzyl, Phenyl oder einen Benzyl- oder Phenylrest, der im Phenylring ein- oder mehrfach durch Halogen, $C_1$-$C_4$-Alkyl, $C_1$-$C_4$-Alkoxy, $CF_3$, ($C_1$-$C_4$-Alkoxy)-carbonyl oder Nitro substituiert ist oder

einen Rest der Formel

$-CR^6H-CO_2R^7$

worin $R^6$ und $R^7$ gleich oder verschieden sind und jeweils H, $C_1$-$C_4$-Alkyl, Phenyl oder Benzyl bedeuten,
$R^2$ $C_1$-$C_4$-Alkyl, das unsubstituiert ist oder ein- oder mehrfach durch Halogen oder durch ($C_1$-$C_4$-Alkoxyl)-carbonyl, Phenyloxycarbonyl, Benzyloxycarbonyl oder Phenyl substituiert ist,
$R^3$ H, $C_1$-$C_4$-Alkyl oder Allyl, insbesondere H,
$R^4$ einen Rest der Formel

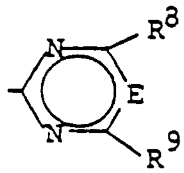 ,

| | |
|---|---|
| $R^8$ und $R^9$ | unabhängig voneinander Halogen, $C_1$-$C_4$-Alkyl, $C_1$-$C_4$-Alkoxy oder einen der letzten beiden vorstehenden Reste, der halogeniert ist, insbesondere die Reste |
| E | CH oder N und |
| Z | O oder S bedeuten. |

**7.** Verfahren zur Bekämpfung von unerwünschtem Pflanzenwuchs, dadurch gekennzeichnet, daß man eine effektive Menge einer der nach einem oder mehreren der Ansprüche 1 bis 6 definierten Verbindung der Formel (I) auf die Pflanzen oder die Anbaufläche appliziert.

**8.** Verfahren zur Pflanzenwachstumsregulierung, dadurch gekennzeichnet, daß es eine Verbindung der Formel (I) nach einem oder mehreren der Ansprüche 1 bis 6 neben inerten Trägerstoffen enthält.

**9.** Verfahren zur Herstellung einer Verbindung der in Anspruch 1 definierten Formel (I), dadurch gekennzeichnet, daß man
(a) eine Verbindung der Formel (II)

$R^1$-Y-A-$NR^2$-$SO_2$-N = C = Z (II),

mit einer Verbindung der Formel (III)

H-$NR^3R^4$ (III),

umsetzt, wobei in den Formeln (II) und (III) A, Y, Z, $R^1$, $R^2$, $R^3$ und $R^4$ die bei Formel (I) angegebenen Bedeutungen haben, oder
(b) eine Verbindung der Formel (IV)

$R^1$-Y-A-$NR^2$-$SO_2$-$NH_2$ (IV)

mit einem Carbamat bzw. Thiocarbamat der Formel (V)

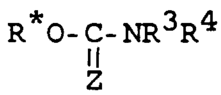 (V)

umsetzt,
wobei in den Formeln (IV) und (V) $R^1$, $R^2$, $R^3$, $R^4$, A, Y und Z die bei Formel (I) angegebenen

Bedeutungen haben und R*$C_1$-$C_6$-Alkyl, $C_1$-$C_4$-Halogenalkyl, Phenyl oder einen Phenylrest bedeutet, der ein- oder mehrfach durch Halogen, $C_1$-$C_4$-Alkyl oder Nitro subtituiert ist, oder

(c) ein Carbamat bzw. Thiocarbamat der Formel (VI) mit einer Verbindung der oben genannten Formel (III)

$$R^1\text{-}Y\text{-}A\text{-}NR^2\text{-}SO_2\text{-}NH\text{-}\underset{\underset{Z}{\|}}{C}\text{-}OR^* \qquad (VI)$$

umsetzt, wobei $R^1$, $R^2$, $R^*$, Y, A und Z die genannten Bedeutungen haben, oder

(d) eine Verbindung der Formel (VII) oder (VIII)

$R^1$-Y-A-NH-$R^2$ (VII)

$R^1$-Y-A-NH-$R^2$ x HCl (VIII)

mit einer Verbindung der Formel (IX)

$$ClSO_2\text{-}NH\text{-}\underset{\underset{Z}{\|}}{C}\text{-}NR^3R^4 \qquad (IX)$$

umsetzt, wobei in den Formeln (VII) bis (IX) A, Y, Z, $R^1$, $R^2$, $R^3$ und $R^4$ die oben angegebenen Bedeutungen haben.

## Claims

### Claims for the following Contracting States : AT, BE, CH, DE, FR, GB, GR, IT, LI, NL

**1.** A compound of the formula (I)

$$R^1\text{-}Y\text{-}A\text{-}NR^2\text{-}SO_2\text{-}NH\text{-}\overset{\overset{Z}{\|}}{C}\text{-}NR^3R^4 \qquad (I)$$

in which

A denotes a saturated or unsaturated, unbranched or branched $C_1$-$C_{10}$-hydrocarbon radical,

$R^1$ denotes $C_1$-$C_8$-alkyl, $C_2$-$C_8$-alkenyl, $C_2$-$C_8$-alkynyl, $C_3$-$C_8$-cycloalkyl, $C_5$-$C_8$-cycloalkenyl or one of the preceding five radicals which is substituted one or more times by halogen or by the radicals selected from the group comprising $C_1$-$C_6$-alkoxy, $C_2$-$C_6$-alkenyloxy, $C_2$-$C_6$-alkynyloxy, $C_1$-$C_6$-alkylthio, $C_1$-$C_6$-alkylsulfinyl, $C_1$-$C_6$-alkylsulfonyl, $C_3$-$C_6$-cycloalkyl, a radical of a three- to six-membered saturated heterocycle having one oxygen atom in the ring, furyl, phenyl and a phenyl radical which is substituted one or more times by radicals from the group comprising halogen, $C_1$-$C_4$-alkyl, $C_1$-$C_4$-alkoxy, $CF_3$, ($C_1$-$C_4$-alkoxy)-carbonyl and nitro, or

$R^1$ denotes phenyl or a phenyl radical which is substituted one or more times by radicals from the group comprising halogen, $C_1$-$C_4$-alkyl, $C_1$-$C_4$-alkoxy, $CF_3$, ($C_1$-$C_4$-alkoxy -carbonyl and nitro, or

$R^1$ denotes a radical of the formula

-CO-$R^5$, -CH$R^6$-COO$R^7$, -CH$R^6$-$CH_2$-COO$R^7$ or -$CH_2$-CH$R^6$-COO$R^7$,

where, in the formulae, $R^5$ represents $C_1$-$C_6$-alkyl, $R^6$ represents hydrogen, $C_1$-$C_4$-

| | |
|---|---|
| | alkyl, phenyl or benzyl and $R^7$ represents H, $C_1$-$C_4$-alkyl, phenyl or benzyl, |
| Y | denotes S or $SO_2$, |
| $R^2$ | denotes H, $C_1$-$C_8$-alkyl, $C_2$-$C_8$-alkenyl, $C_2$-$C_8$-alkynyl or one of the preceding three radicals which is substituted one or more times by halogen or by radicals from the group comprising $C_1$-$C_6$-alkoxy, $C_2$-$C_6$-alkenyloxy, $C_2$-$C_6$-alkynyloxy, $C_1$-$C_6$-alkylthio, $C_1$-$C_6$-alkylsulfinyl, $C_1$-$C_6$-alkylsulfonyl, ($C_1$-$C_6$-alkoxy)-carbonyl, phenoxycarbonyl, benzyloxycarbonyl and phenyl, denotes $C_3$-$C_8$-cycloalkyl which is unsubstituted or substituted one or more times by halogen or once or twice by $C_1$-$C_4$-alkoxy or $C_1$-$C_4$-alkylthio, denotes $C_5$-$C_8$-cycloalkenyl, cyclopropylmethyl, epoxypropyl, furfuryl, tetrahydrofurfuryl, phenoxy-$C_1$-$C_6$-alkyl, phenyl or one of the last two preceding radicals which is substituted in the phenyl ring by halogen, $C_1$-$C_4$-alkyl, $C_1$-$C_4$-alkoxy or nitro, |
| $R^3$ | denotes H, $C_1$-$C_8$-alkyl, $C_2$-$C_8$-alkenyl, $C_2$-$C_8$-alkynyl or $C_1$-$C_4$-alkoxy, |
| $R^4$ | denotes a radical of the formula |

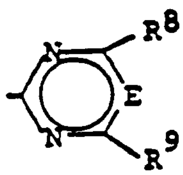 , 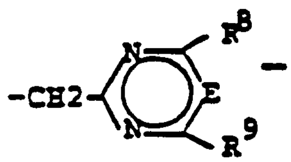 , 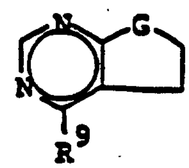 ,

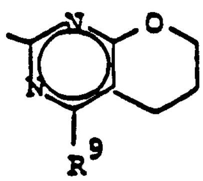 , 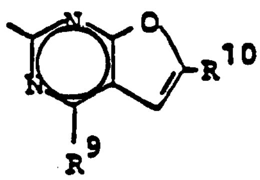 , 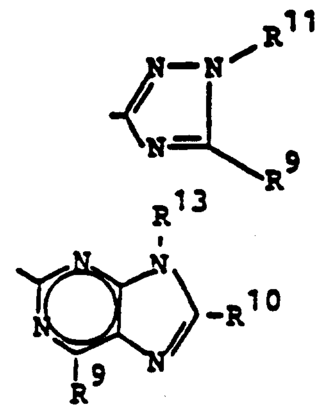 ,

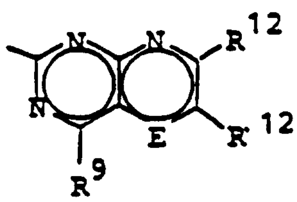 or

| | |
|---|---|
| $R^8$ and $R^9$ | denote, independently of one another, H, halogen, $C_1$-$C_6$-alkyl, $C_1$-$C_6$-alkoxy, $C_1$-$C_6$-alkylthio or one of the last three preceding radicals which is substituted one or more times by halogen or once or twice by $C_1$-$C_4$-alkoxy or $C_1$-$C_4$-alkylthio, or denote a radical $NR^{14}R^{15}$, $C_3$-$C_6$-cycloalkyl, $-OCHR^{16}COOR^{17}$, $C_3$-$C_5$-alkenyl, $C_2$-$C_4$-alkynyl, $C_3$-$C_5$-alkenyloxy or $C_3$-$C_5$-alkynyloxy, |
| $R^{10}$ | denotes hydrogen or $C_1$-$C_4$-alkyl, |
| $R^{11}$ | denotes $C_1$-$C_4$-alkyl, $-CHF_2$ or $-CH_2CF_3$, |
| $R^{12}$ | denote, independently of one another, H, $C_1$-$C_4$-alkyl, $C_1$-$C_4$-alkoxy or halogen, |
| $R^{13}$ | denotes hydrogen, $C_1$-$C_4$-alkyl, $CHF_2$ or $CH_2CF_3$, |
| $R^{14}$ and $R^{15}$ | denote, independently of one another, H, $C_1$-$C_4$-alkyl, $C_2$-$C_4$-alkenyl or $C_3$-$C_4$-alkynyl, |
| $R^{16}$ | denotes hydrogen or $C_1$-$C_4$-alkyl, |
| $R^{17}$ | denotes hydrogen or $C_1$-$C_4$-alkyl, |
| E | denotes CH or N, |
| G | denotes $CH_2$ or O and |
| Z | denotes O or S, as well as the salts thereof. |

**2.** A compound as claimed in claim 1, wherein

$R^1$ denotes $C_1$-$C_4$-alkyl, a $C_1$-$C_4$-alkyl radical which is substituted one or more times by halogen or once or twice by $C_1$-$C_4$-alkoxy, $C_2$-$C_3$-alkenyloxy, $C_2$-$C_3$-alkynyloxy, $C_1$-$C_4$-alkylthio, $C_1$-

$C_4$-alkylsulfinyl, $C_1$-$C_4$-alkylsulfonyl, phenyl or a phenyl radical which is substituted one to three times by radicals from the group comprising halogen, $C_1$-$C_4$-alkyl, $C_1$-$C_4$-alkoxy, $CF_3$, ($C_1$-$C_4$-alkoxy)-carbonyl and nitro, or denotes $C_3$-$C_8$-cycloalkyl or a $C_3$-$C_8$-cycloalkyl radical which is substituted one or more times by halogen or once or twice by $C_1$-$C_4$-alkoxy or $C_1$-$C_4$-alkyl-thio, or denotes $C_5$-$C_8$-cycloalkenyl, cyclopropylmethyl, epoxypropyl, furfuryl, tetrahydrofurfuryl, benzyl, phenyl or a benzyl or phenyl radical which is substituted in the phenyl ring by one or more radicals from the group comprising halogen, $C_1$-$C_4$-alkyl, $C_1$-$C_4$-alkoxy, $CF_3$, ($C_1$-$C_4$-alkoxy)-carbonyl and nitro, or denotes a radical of the formula $-COR^5$, $CHR^6-COOR^7$, $CHR^6-CH_2-COOR^7$ or $CH_2-CHR^6-COOR^7$,

$R^5$ denotes $C_1$-$C_4$-alkyl,

$R^6$ denotes H, $C_1$-$C_4$-alkyl, benzyl or phenyl and

$R^7$ denotes H, $C_1$-$C_4$-alkyl, benzyl or phenyl.

**3.** A compound as claimed in claim 1 or 2, wherein

$R^2$ denotes $C_1$-$C_4$-alkyl, a $C_1$-$C_4$-alkyl radical which is substituted one or more times by halogen or once or twice by $C_1$-$C_4$-alkoxy, $C_2$-$C_4$-alkenyloxy, propargyloxy, $C_1$-$C_4$-alkylthio, $C_1$-$C_4$-alkylsulfinyl, $C_1$-$C_4$-alkylsulfonyl, ($C_1$-$C_4$-alkoxy)-carbonyl, phenoxycarbonyl, benzyloxycarbonyl or phenyl, or denotes $C_3$-$C_8$-cycloalkyl.

**4.** A compound as claimed in one or more of claims 1 to 3, wherein

$R^4$ denotes a radical of the formula

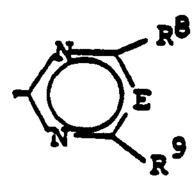

,

and

$R^8$ and $R^9$ denote, independently of one another, halogen, $C_1$-$C_4$-alkyl, $C_1$-$C_4$-alkoxy, $C_1$-$C_4$-alkylthio or one of the last three preceding radicals which are substituted one or more times by halogen or once or twice by $C_1$-$C_4$-alkoxy, $C_1$-$C_4$-alkylthio, or denote a radical $NR^{14}R^{15}$, $C_3$-$C_6$-cycloalkyl, $-OCHR^{16}COOR^{17}$, allyl, propargyl, allyloxy or propargyloxy,

$R^{14}$ and $R^{15}$ denote, independently of one another, H or $C_1$-$C_4$-alkyl,

$R^{16}$ denotes H or $C_1$-$C_4$-alkyl,

$R^{17}$ denotes $C_1$-$C_4$-alkyl and

E denotes CH or N.

**5.** A compound as claimed in one or more of claims 1 to 4, wherein

A denotes a radical of the formula $CH_2$, $CH_2CH_2$, CHR, CRR', $CH_2CHR$ or $CH_2CRR'$, where R and R' denote, independently of one another, $C_1$-$C_4$-alkyl or $C_2$-$C_4$-alkenyl.

**6.** A compound as claimed in one or more of claims 1 to 5, wherein

A denotes a radical of the formula $-CH_2-$ or $-CH_2-CH_2-$,

$R^1$ denotes $C_1$-$C_4$-alkyl, a $C_1$-$C_4$-alkyl radical which is substituted one or more times by halogen or once or twice by $C_1$-$C_4$-alkoxy, denotes $C_3$-$C_8$-cycloalkyl which is substituted one or more times by halogen or Is unsubstituted, denotes benzyl, phenyl or a benzyl or phenyl radical which is substituted in the phenyl ring one or more times by halogen, $C_1$-$C_4$-alkyl, $C_1$-$C_4$-alkoxy, $CF_3$, ($C_1$-$C_4$-alkoxy)-carbonyl or nitro, or denotes a radical of the formula

$$-CR^6H-CO_2R^7$$

in which $R^6$ and $R^7$ are identical or different and each denotes H, $C_1$-$C_4$-alkyl, phenyl or benzyl,

$R^2$ denotes $C_1$-$C_4$-alkyl which is unsubstituted or substituted one or more times by halogen or by ($C_1$-$C_4$-alkoxy)-carbonyl, phenyloxycarbonyl, benzyloxycarbonyl or phenyl,

$R^3$ denotes H, $C_1$-$C_4$-alkyl or allyl, especially H,

$R^4$ denotes a radical of the formula

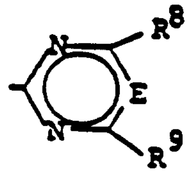 ,

$R^8$ and $R^9$ denote, independently of one another, halogen, $C_1$-$C_4$-alkyl, $C_1$-$C_4$-alkoxy or one of the last two preceding radicals which is halogenated,

E denotes CH or N and

Z denotes O or S.

**7.** A herbicidal agent which contains a compound of the formula (I) or one of its salts as claimed in one or more of claims 1 to 6 in addition to inert carriers.

**8.** A plant-growth regulating agent which contains a compound of the formula (I) or one of its salts as claimed in at least one of claims 1 to 6 in addition to inert carriers.

**9.** The use of compounds of the formula (I) and of one of their salts as claimed in one or more of claims 1 to 6 as herbicides or plant-growth regulators.

**10.** A process for the preparation of a compound of the formula (I) as claimed in claim 1, which comprises
(a) reacting a compound of the formula (II)

$R^1$-Y-A-$NR^2$-$SO_2$-N=C=Z (II)

with a compound of the formula (III)

H-$NR^3R^4$ (III)

where in formulae (II) and (III) A, Y, Z, $R^1$, $R^2$, $R^3$ and $R^4$ have the meanings specified for formula (I), or
(b) reacting a compound of the formula (IV)

$R^1$-Y-A-$NR^2$-$SO_2$-$NH_2$ (IV)

with a carbamate or thiocarbamate of the formula (V)

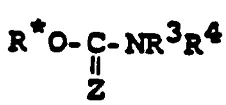 (V)

where in the formulae (IV) and (V) $R^1$, $R^2$, $R^3$, $R^4$, A, Y and Z have the meanings specified for formula (I), and $R^*$ denotes $C_1$-$C_6$-alkyl, $C_1$-$C_4$-halogenoalkyl, phenyl or a phenyl radical which is substituted one or more times by halogen, $C_1$-$C_4$-alkyl or nitro, or
(c) reacting a carbamate or thiocarbamate of the formula (VI) with a compound of the abovementioned formula (III)

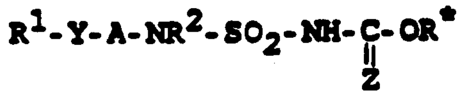

(VI)

where $R^1$, $R^2$, $R^*$, Y, A and Z have the said meanings, or

(d) reacting a compound of the formula (VII) or (VIII)

$R^1$-Y-A-NH-$R^2$ (VII)

$R^1$-Y-A-NH-$R^2$ (VIII)

with a compound of the formula (IX)

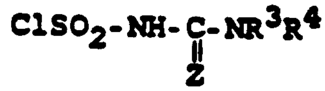

(IX)

where in the formulae (VII) to (IX) A, Y, Z, $R^1$, $R^2$, $R^3$ and $R^4$ have the meanings specified above.

**Claims for the following Contracting State : ES**

**1.** The use of a compound of the formula (I)

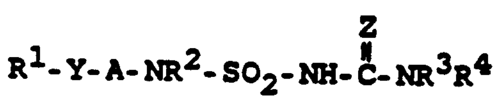

(I)

in which

A denotes a saturated or unsaturated, unbranched or branched $C_1$-$C_{10}$-hydrocarbon radical,

$R^1$ denotes $C_1$-$C_8$-alkyl, $C_2$-$C_8$-alkenyl, $C_2$-$C_8$-alkynyl, $C_3$-$C_8$-cycloalkyl, $C_5$-$C_8$-cycloalkenyl or one of the preceding five radicals which is substituted one or more times by halogen or by the radicals selected from the group comprising $C_1$-$C_6$-alkoxy, $C_2$-$C_6$-alkenyloxy, $C_2$-$C_6$-alkynyloxy, $C_1$-$C_6$-alkylthio, $C_1$-$C_6$-alkylsulfinyl, $C_1$-$C_6$-alkylsulfonyl, $C_3$-$C_6$-cycloalkyl, a radical of a three- to six-membered saturated heterocycle having one oxygen atom in the ring, furyl, phenyl and a phenyl radical which is substituted one or more times by radicals from the group comprising halogen, $C_1$-$C_4$-alkyl, $C_1$-$C_4$-alkoxy, $CF_3$, ($C_1$-$C_4$-alkoxy)-carbonyl and nitro, or

$R^1$ denotes phenyl or a phenyl radical which is substituted one or more times by radicals from the group comprising halogen, $C_1$-$C_4$-alkyl, $C_1$-$C_4$-alkoxy, $CF_3$, ($C_1$-$C_4$-alkoxy)-carbonyl and nitro, or

$R^1$ denotes a radical of the formula

$-CO-R^5$, $-CHR^6-COOR^7$, $-CHR^6-CH_2-COOR^7$ or $-CH_2-CHR^6-COOR^7$,

where, in the formulae, $R^5$ represents $C_1$-$C_6$-alkyl, $R^6$ represents hydrogen, $C_1$-$C_4$-alkyl, phenyl or benzyl and $R^7$ represents H, $C_1$-$C_4$-alkyl, phenyl or benzyl,

Y denotes S or $SO_2$,

$R^2$ denotes H, $C_1$-$C_8$-alkyl, $C_2$-$C_8$-alkenyl, $C_2$-$C_8$-alkynyl or one of the preceding three radicals which is substituted one or more times by halogen or by radicals from the group comprising $C_1$-$C_6$-alkoxy, $C_2$-$C_6$-alkenyloxy, $C_2$-$C_6$-alkynyloxy, $C_1$-$C_6$-alkylthio, $C_1$-$C_6$-alkylsulfinyl, $C_1$-$C_6$-alkylsulfonyl, ($C_1$-$C_6$-alkoxy)-carbonyl, phenoxycarbonyl, benzyloxycarbonyl and phenyl, denotes $C_3$-$C_8$-cycloalkyl which is unsubstituted or substituted one or more times by halogen or once or twice by $C_1$-$C_4$-

alkoxy or $C_1$-$C_4$-alkylthio, denotes $C_5$-$C_8$-cycloalkenyl, cyclopropylmethyl, epoxypropyl, furfuryl, tetrahydrofurfuryl, phenoxy-$C_1$-$C_6$-alkyl, phenyl or one of the last two preceding radicals which is substituted in the phenyl ring by halogen, $C_1$-$C_4$-alkyl, $C_1$-$C_4$-alkoxy or nitro,

$R^3$ denotes H, $C_1$-$C_8$-alkyl, $C_2$-$C_8$-alkenyl, $C_2$-$C_8$-alkynyl or $C_1$-$C_4$-alkoxy,

$R^4$ denotes a radical of the formula

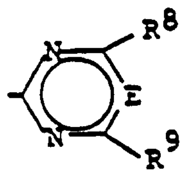 , 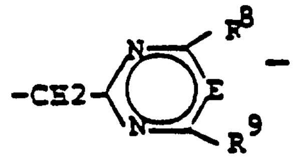 , 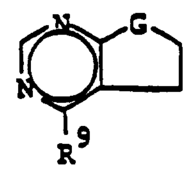 ,

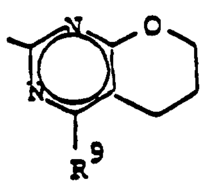 , 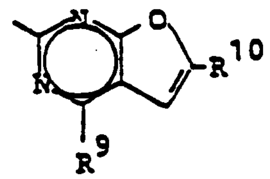 , 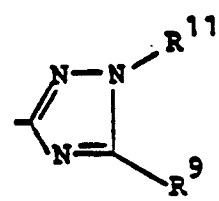 ,

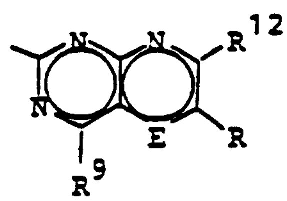 or 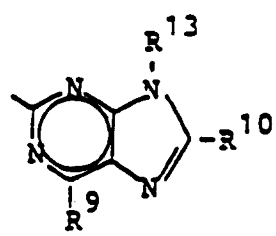

$R^8$ and $R^9$ denote, independently of one another, H, halogen, $C_1$-$C_6$-alkyl, $C_1$-$C_6$-alkoxy, $C_1$-$C_6$-alkylthio or one of the last three preceding radicals which is substituted one or more times by halogen or once or twice by $C_1$-$C_4$-alkoxy or $C_1$-$C_4$-alkylthio, or denote a radical $NR^{14}R^{15}$, $C_3$-$C_6$-cycloalkyl, $-OCHR^{16}COOR^{17}$, $C_3$-$C_5$-alkenyl, $C_2$-$C_4$-alkynyl, $C_3$-$C_5$-alkenyloxy or $C_3$-$C_5$-alkynyloxy,

$R^{10}$ denotes hydrogen or $C_1$-$C_4$-alkyl,

$R^{11}$ denotes $C_1$-$C_4$-alkyl, $-CHF_2$ or $-CH_2CF_3$,

$R^{12}$ denote, independently of one another, H, $C_1$-$C_4$-alkyl, $C_1$-$C_4$-alkoxy or halogen,

$R^{13}$ denotes hydrogen, $C_1$-$C_4$-alkyl, $CHF_2$ or $CH_2CF_3$,

$R^{14}$ and $R^{15}$ denote, independently of one another, H, $C_1$-$C_4$-alkyl, $C_2$-$C_4$-alkenyl or $C_3$-$C_4$-alkynyl,

$R^{16}$ denotes hydrogen or $C_1$-$C_4$-alkyl,

$R^{17}$ denotes hydrogen or $C_1$-$C_4$-alkyl,

E denotes CH or N,

G denotes $CH_2$ or O and

Z denotes O or S, as well as the salts thereof as herbicides or plant-growth regulators.

**2.** The use as claimed in claim 1, wherein

$R^1$ denotes $C_1$-$C_4$-alkyl, a $C_1$-$C_4$-alkyl radical which is substituted one or more times by halogen or once or twice by $C_1$-$C_4$-alkoxy, $C_2$-$C_3$-alkenyloxy, $C_2$-$C_3$-alkynyloxy, $C_1$-$C_4$-alkylthio, $C_1$-$C_4$-alkylsulfinyl, $C_1$-$C_4$-alkylsulfonyl, phenyl or a phenyl radical which is substituted one to three times by radicals from the group comprising halogen, $C_1$-$C_4$-alkyl, $C_1$-$C_4$-alkoxy, $CF_3$, ($C_1$-$C_4$-alkoxy)-carbonyl and nitro, or denotes $C_3$-$C_8$-cycloalkyl or a $C_3$-$C_8$-cycloalkyl radical which is substituted one or more times by halogen or once or twice by $C_1$-$C_4$-alkoxy or $C_1$-$C_4$-alkylthio, or denotes $C_5$-$C_8$-cycloalkenyl, cyclopropylmethyl, epoxypropyl, furfuryl, tetrahydrofurfuryl, benzyl, phenyl or a benzyl or phenyl radical which is substituted in the phenyl ring by one or more radicals from the group comprising halogen, $C_1$-$C_4$-alkyl, $C_1$-$C_4$-alkoxy, $CF_3$, ($C_1$-$C_4$-alkoxy)-carbonyl and nitro, or denotes a radical of the formula

-$COR^5$, $CHR^6$-$COOR^7$, $CHR^6$-$CH_2$-$COOR^7$ or $CH_2$-$CHR^6$-$COOR^7$,

$R^5$ denotes $C_1$-$C_4$-alkyl,

$R^6$ denotes H, $C_1$-$C_4$-alkyl, benzyl or phenyl and

$R^7$ denotes H, $C_1$-$C_4$-alkyl, benzyl or phenyl.

**3.** The use as claimed in claim 1 or 2, wherein

$R^2$ denotes $C_1$-$C_4$-alkyl, a $C_1$-$C_4$-alkyl radical which is substituted one or more times by halogen or once or twice by $C_1$-$C_4$-alkoxy, $C_2$-$C_4$-alkenyloxy, propargyloxy, $C_1$-$C_4$-alkylthio, $C_1$-$C_4$-alkylsulfinyl, $C_1$-$C_4$-alkylsulfonyl, ($C_1$-$C_4$-alkoxy)-carbonyl, phenoxycarbonyl, benzyloxycarbonyl or phenyl, or denotes $C_3$-$C_8$-cycloalkyl.

**4.** The use as claimed in one or more of claims 1 to 3, wherein

$R^4$ denotes a radical of the formula

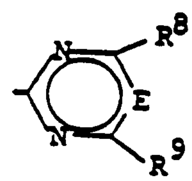

,

and

$R^8$ and $R^9$ denote, independently of one another, halogen, $C_1$-$C_4$-alkyl, $C_1$-$C_4$-alkoxy, $C_1$-$C_4$-alkylthio or one of the last three preceding radicals which are substituted one or more times by halogen or once or twice by $C_1$-$C_4$-alkoxy, $C_1$-$C_4$-alkylthio, or denote a radical $NR^{14}R^{15}$, $C_3$-$C_6$-cycloalkyl, -$OCHR^{16}COOR^{17}$, allyl, propargyl, allyloxy or propargyloxy,

$R^{14}$ and $R^{15}$ denote, independently of one another, H or $C_1$-$C_4$-alkyl,

$R^{16}$ denotes H or $C_1$-$C_4$-alkyl,

$R^{17}$ denotes $C_1$-$C_4$-alkyl and

E denotes CH or N.

**5.** The use as claimed in one or more of claims 1 to 4, wherein

A denotes a radical of the formula $CH_2$, $CH_2CH_2$, CHR, CRR', $CH_2CHR$ or $CH_2CRR'$, where R and R' denote, independently of one another, $C_1$-$C_4$-alkyl or $C_2$-$C_4$-alkenyl.

**6.** A compound as claimed in one or more of claims 1 to 5, wherein

A denotes a radical of the formula -$CH_2$- or -$CH_2$-$CH_2$-,

$R^1$ denotes $C_1$-$C_4$-alkyl, a $C_1$-$C_4$-alkyl radical which is substituted one or more times by halogen or once or twice by $C_1$-$C_4$-alkoxy, denotes $C_3$-$C_8$-cycloalkyl which is substituted one or more times by halogen or is unsubstituted, denotes benzyl, phenyl or a benzyl or phenyl radical which is substituted in the phenyl ring one or more times by halogen, $C_1$-$C_4$-alkyl, $C_1$-$C_4$-alkoxy, $CF_3$, ($C_1$-$C_4$-alkoxy)-carbonyl or nitro, or denotes a radical of the formula

-$CR^6H$-$CO_2R^7$

in which $R^6$ and $R^7$ are identical or different and each denotes H, $C_1$-$C_4$-alkyl, phenyl or benzyl,

$R^2$ denotes $C_1$-$C_4$-alkyl which is unsubstituted or substituted one or more times by halogen or by ($C_1$-$C_4$-alkoxy)-carbonyl, phenyloxycarbonyl, benzyloxycarbonyl or phenyl,

$R^3$ denotes H, $C_1$-$C_4$-alkyl or allyl, especially H,

$R^4$ denotes a radical of the formula

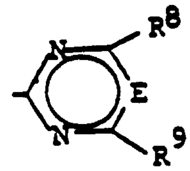 ,

| | |
|---|---|
| $R^8$ and $R^9$ | denote, independently of one another, halogen, $C_1$-$C_4$-alkyl, $C_1$-$C_4$-alkoxy or one of the last two preceding radicals which is halogenated, |
| E | denotes CH or N and |
| Z | denotes O or S. |

**7.** A method for controlling unwanted plant growth, which comprises applying an effective amount of a compound of the formula (I) defined in one or more of claims 1 to 6 to the plants or the cultivated area.

**8.** A method for regulating plant growth, which contains a compound of the formula (I) as claimed in one or more of claims 1 to 6 in addition to inert carriers.

**9.** A process for the preparation of a compound of the formula (I) defined in claim 1, which comprises

(a) reacting a compound of the formula (II)

$R^1$-Y-A-$NR^2$-$SO_2$-N = C = Z (II)

with a compound of the formula (III)

H-$NR^3R^4$ (III)

where in formulae (II) and (III) A, Y, Z, $R^1$, $R^2$, $R^3$ and $R^4$ have the meanings specified for formula (I), or

(b) reacting a compound of the formula (IV)

$R^1$-Y-A-$NR^2$-$SO_2$-$NH_2$ (IV)

with a carbamate or thiocarbamate of the formula (V)

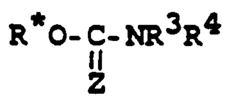 (V)

where in the formulae (IV) and (V) $R^1$, $R^2$, $R^3$, $R^4$, A, Y and Z have the meanings specified for formula (I), and R* denotes $C_1$-$C_6$-alkyl, $C_1$-$C_4$-halogenoalkyl, phenyl or a phenyl radical which is substituted one or more times by halogen, $C_1$-$C_4$-alkyl or nitro, or

(c) reacting a carbamate or thiocarbamate of the formula (VI) with a compound of the abovementioned formula (III)

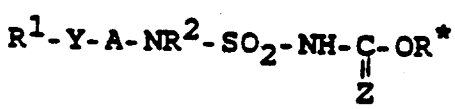 (VI)

where $R^1$, $R^2$, R*, Y, A and Z have the said meanings, or

(d) reacting a compound of the formula (VII) or (VIII)

$R^1$-Y-A-NH-$R^2$ (VII)

$R^1$-Y-A-NH-$R^2$ x HCL (VIII)

with a compound of the formula (IX)

$$ClSO_2\text{-}NH\text{-}\underset{\underset{Z}{\|}}{C}\text{-}NR^3R^4 \qquad (IX)$$

where in the formulae (VII) to (IX) A, Y, Z, $R^1$, $R^2$, $R^3$ and $R^4$ have the meanings specified above.

## Revendications

## Revendications pour les Etats contractants suivants : AT, BE, CH, DE, FR, GB, GR, IT, LI, NL

**1.** Composé de formule générale (I)

$$R^1\text{-}Y\text{-}A\text{-}NR^2\text{-}SO_2\text{-}NH\text{-}\overset{\overset{Z}{\|}}{C}\text{-}NR^3R^4 \qquad (I)$$

dans laquelle

A est un radical hydrocarboné en $C_1$-$C_{10}$, saturé ou insaturé, à chaîne droite ou ramifiée, de préférence un radical de formule $CH_2$, $CH_2CH_2$, CHR, CRR', $CH_2CHR$ ou $CH_2CRR'$, où R et R', indépendamment l'un de l'autre, sont chacun un radical alkyle en $C_1$-$C_4$ ou alcényle en $C_2$-$C_4$.

$R^1$ est un radical alkyle en $C_1$-$C_8$, alcényle en $C_2$-$C_8$, alcynyle en $C_2$-$C_8$, cycloalkyle en $C_3$-$C_8$, cycloalcényle en $C_5$-$C_8$, ou encore l'un des 5 radicaux ci-dessus, substitué une ou plusieurs fois par des halogènes ou par des radicaux qui sont choisis parmi l'ensemble comprenant les substituants alcoxy en $C_1$-$C_6$, alcényloxy en $C_2$-$C_6$, alcynyloxy en $C_2$-$C_6$, alkylthio en $C_1$-$C_6$, alkylsulfinyle en $C_1$-$C_6$, alkylsulfonyle en $C_1$-$C_6$, cycloalkyle en $C_3$-$C_6$, le résidu d'un hétérocycle saturé ayant de 3 à 6 chaînons, avec un atome d'oxygène dans le noyau, ou encore les radicaux furyle, phényle, et les radicaux phényle une ou plusieurs substitués par des substituants choisis parmi l'ensemble comprenant les halogènes et les radicaux alkyle en $C_1$-$C_4$, alcoxy en $C_1$-$C_4$, $CF_3$, (alcoxy en $C_1$-$C_4$)-carbonyle et nitro, ou bien

$R^1$ est le radical phényle ou est un radical phényle une ou plusieurs fois substitué par des radicaux choisis parmi l'ensemble comprenant les halogènes et les radicaux alkyle en $C_1$-$C_4$, alcoxy en $C_1$-$C_4$, $CF_3$, (alcoxy en $C_1$-$C_4$)-carbonyle et nitro, ou bien

$R^1$ est un radical de formule

$-CO-R^5$, $-CHR^6-COOR^7$, $-CHR^6-CH_2-COOR^7$ ou $-CH_2-CHR^6-COOR^7$,

où, dans les formules, $R^5$ est un radical alkyle en $C_1$-$C_6$, $R^6$ est un hydrogène, un radical alkyle en $C_1$-$C_4$, le radical phényle ou benzyle, et $R^7$ est H ou un radical alkyle en $C_1$-$C_4$ ou le radical phényle ou benzyle,

Y est S ou $SO_2$,

$R^2$ est H ou un radical alkyle en $C_1$-$C_8$, alcényle en $C_2$-$C_8$, alcynyle en $C_2$-$C_8$ ou l'un des trois radicaux ci-dessus, qui sont une ou plusieurs fois substitué par des halogènes ou par des substituants choisis parmi l'ensemble comprenant les radicaux alcoxy en $C_1$-$C_6$, alcényloxy en $C_2$-$C_6$, alcynyloxy en $C_2$-$C_6$, alkylthio en $C_1$-$C_6$, alkylsulfinyle en $C_1$-$C_6$, alkylsulfonyle en $C_1$-$C_6$, (alcoxy en $C_1$-$C_6$)-carbonyle, phénoxycarbonyle, benzyloxycarbonyle et phényle, ou bien encore représente un radical cycloalkyle en $c_3$-$c_8$, lequel est non substitué ou est une ou plusieurs fois substitué par des halogènes ou une ou deux fois par des radicaux alcoxy en $C_1$-$C_4$ ou alkylthio en $C_1$-$C_4$ ; cycloalcényle en $C_5$-$C_8$, cyclopropylméthyle, époxypropyle, furfuryle, tétrahydrofurfuryle, phénoxy(alkyle en $C_1$-$C_6$), phényle, ou encore représente l'un des deux derniers radicaux ci-dessus, lequel est substitué sur le noyau phényle par des substituants halogéno, alkyle en $C_1$-$C_4$, alcoxy en $C_1$-$C_4$ ou nitro,

$R^3$ est H ou un radical alkyle en $C_1$-$C_8$, alcényle en $C_2$-$C_8$, alcynyle en $C_2$-$C_8$, ou alcoxy en $C_1$-$C_4$,

$R^4$ est un radical de formule

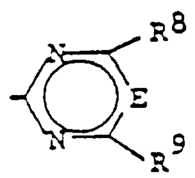 , 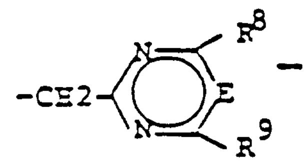 , 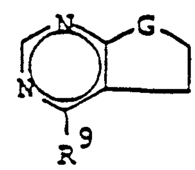 ,

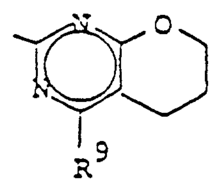 , 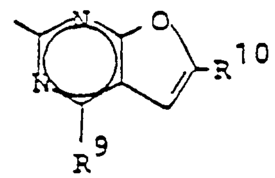 , 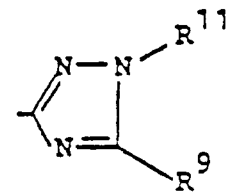 ,

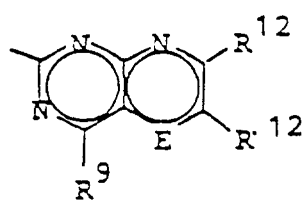 ou 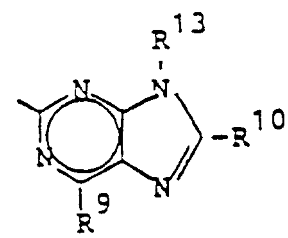

$R^8$ et $R^9$, indépendamment l'un de l'autre, sont chacun H ou un radical halogéno, alkyle en $C_1$-$C_6$, alcoxy en $C_1$-$C_6$, alkylthio en $C_1$-$C_6$, ou encore l'un des trois derniers radicaux ci-dessus, qui sont une ou plusieurs fois substitué par des radicaux halogéno ou une ou deux fois par des substituants alcoxy en $C_1$-$C_4$ ou alkylthio en $C_1$-$C_4$, ou encore un radical $NR^{14}R^{15}$, cycloalkyle en $C_3$-$C_6$, $-OCHR^{16}COOR^{17}$, alcényle en $C_3$-$C_5$, alcynyle en $C_2$-$C_4$, alcényloxy en $C_3$-$C_5$ ou alcynyloxy en $C_3$-$C_5$,

$R^{10}$ est un hydrogène ou un radical alkyle en $C_1$-$C_4$,

$R^{11}$ est un radical alkyle en $C_1$-$C_4$, $-CHF_2$ ou $-CH_2CF_3$,

les radicaux $R^{12}$, indépendamment l'un de l'autre, sont chacun H ou un radical alkyle en $C_1$-$C_4$, alcoxy en $C_1$-$C_4$ ou halogéno,

$R^{13}$ est un hydrogène ou un radical alkyle en $C_1$-$C_4$,$CHF_2$ ou $CH_2CF_3$,

$R^{14}$ et $R^{15}$, indépendamment l'un de l'autre, sont chacun H ou un radical alkyle en $C_1$-$C_4$, alcényle en $C_2$-$C_4$ ou alcynyle en $C_3$-$C_4$,

$R^{16}$ est un hydrogène ou un radical alkyle en $C_1$-$C_4$,

$R^{17}$ est un hydrogène ou un radical alkyle en $C_1$-$C_4$,

E est CH ou N,

G est $CH_2$ ou O, et

Z est O ou S, ainsi que leurs sels.

**2.** Composé selon la revendication 1, caractérisé en ce que :

$R^1$ est un radical alkyle en $C_1$-$C_4$, un radical alkyle en $C_1$-$C_4$ une ou plusieurs fois substitué par des halogènes ou une ou deux fois substitués par des radicaux alcoxy en $C_1$-$C_4$, alcényloxy en $C_2$-$C_3$, alcynyloxy en $C_2$-$C_3$, alkylthio en $C_1$-$C_4$, alkylsulfinyle en $C_1$-$C_4$, alkylsulfonyle en $C_1$-$C_4$, phényle, ou encore par un radical phényle une à trois fois substitués par des substituants choisis parmi l'ensemble comprenant les halogènes et les radicaux alkyle en $C_1$-$C_4$, alcoxy en $C_1$-$C_4$, $CF_3$, (alcoxy en $C_1$-$C_4$)-carbonyle et nitro ; ou bien encore cycloalkyle en $C_3$-$C_8$, ou un radical cycloalkyle en $C_3$-$C_8$ une ou plusieurs fois substitué par des halogènes ou une ou deux fois substitués par des substituants alcoxy en $C_1$-$C_4$ ou alkylthio en $C_1$-$C_4$ ; ou bien cycloalcényle en $C_5$-$C_8$, cyclopropylméthyle, époxypropyle, furfuryle, tétrahydrofurfuryle, benzyle, phényle, ou encore un résidu benzyle ou phényle substitué dans le noyau phényle par un ou plusieurs radicaux choisis parmi l'ensemble comprenant les halogènes et

les radicaux alkyle en $C_1$-$C_4$, alcoxy en $C_1$-$C_4$, $CF_3$, (alcoxy en $C_1$-$C_4$)-carbonyle et nitro ; ou bien un radical de formule -$COR^5$, $CHR^6$-$COOR^7$, $CHR^6$-$CH_2$-$COOR^7$ ou $CH_2$-$CHR^6$-$COOR^7$,

$R^5$ est un radical alkyle en $C_1$-$C_4$,

$R^6$ est H ou un radical alkyle en $C_1$-$C_4$, benzyle ou phényle,

$R^7$ est H ou radical alkyle en $C_1$-$C_4$, benzyle ou phényle.

**3.** Composé selon la revendication 1 ou 2, caractérisé en ce que :

$R^2$ est un radical alkyle en $C_1$-$C_4$, un radical alkyle en $C_1$-$C_4$ une ou plusieurs fois substitué par des halogènes ou une ou deux fois par des substituants alcoxy en $C_1$-$C_4$, alcényloxy en $C_1$-$C_4$, propargyloxy, alkylthio en $C_1$-$C_4$, alkylsulfinyle en $C_1$-$C_4$, alkylsulfonyle en $C_1$-$C_4$, (alcoxy en $C_1$-$C_4$)-carbonyle, phénoxycarbonyle, benzyloxycarbonyle ou phényle, ou encore est un radical cycloalkyle en $C_3$-$C_8$.

**4.** Composé selon l'une ou plusieurs des revendications 1 à 3 caractérisé en ce que :

$R^4$ est un radical de formule

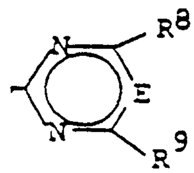

,

et

les radicaux $R^8$ et $R^9$, indépendamment l'un de l'autre, sont chacun un halogène ou un radical alkyle en $C_1$-$C_4$, alcoxy en $C_1$-$C_4$, alkylthio en $C_1$-$C_4$ ou l'un des trois derniers radicaux ci-dessus, qui sont substitués une ou plusieurs fois par des halogènes ou une ou deux fois par des substituants alcoxy ou alkylthio en $C_1$-$C_4$, ou encore un radical $NR^{14}R^{15}$, cycloalkyle en $C_3$-$C_6$, -$OCHR^{16}COOR^{17}$, allyle, propargyle, allyloxy ou propargyloxy,

$R^{14}$ et $R^{15}$, indépendamment l'un de l'autre, sont chacun H ou un radical alkyle en $C_1$-$C_4$,

$R^{16}$ est H ou un radical alkyle en $C_1$-$C_4$,

$R^{17}$ est un radical alkyle en $C_1$-$C_4$, et

E est CH ou N.

**5.** Composé selon l'une ou plusieurs des revendications 1 à 4, caractérisé en ce que :

A est un radical de formule $CH_2$, $CH_2CH_2$, CHR, CRR', $CH_2CHR$ ou $CH_2CRR'$, où R et R', indépendamment l'un de l'autre, sont chacun un radical alkyle en $C_1$-$C_4$ ou alcényle en $C_2$-$C_4$.

**6.** Composé selon l'une ou plusieurs des revendications 1 à 5, caractérisé en ce que :

A est un radical de formule -$CH_2$- ou -$CH_2$-$CH_2$-,

$R^1$ est un radical alkyle en $C_1$-$C_4$, un radical alkyle en $C_1$-$C_4$ une ou plusieurs fois substitué par des halogènes ou une ou deux fois par des substituants alcoxy en $C_1$-$C_4$ ; cycloalkyle en $C_3$-$C_8$, une ou plusieurs fois substitué par des halogènes, ou non substitué ; benzyle, phényle, ou encore un radical benzyle ou phényle substitué sur le noyau phényle une ou plusieurs fois par des halogènes ou des substituants alkyle en $C_1$-$C_4$, alcoxy en $C_1$-$C_4$, $CF_3$, (alcoxy en $C_1$-$C_4$)-carbonyle ou nitro ; ou bien encore un radical de formule

-$CR^6H$-$CO_2R^7$

où $R^6$ et $R^7$ sont identiques ou différents et représentent chacun H ou un radical alkyle en $C_1$-$C_4$, phényle ou benzyle,

$R^2$ est un radical alkyle en $C_1$-$C_4$, qui est non substitué ou est substitué une ou plusieurs fois par des halogènes ou par des radicaux (alcoxy en $C_1$-$C_4$) -carbonyle, phényloxycarbonyle, benzyloxycarbonyle ou phényle,

$R^3$ est H ou un radical alkyle en $C_1$-$C_4$ ou allyle, en particulier H,

$R^4$ est un radical de formule

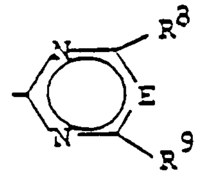 ,

$R^8$ et $R^9$, indépendamment l'un de l'autre, sont chacun un halogène ou un radical alkyle en $C_1$-$C_4$, alcoxy en $C_1$-$C_4$ ou l'un des deux derniers radicaux ci-dessus, lequel est halogéné, en particulier les radicaux $CH_3$, $OCH_3$, $OC_2H_5$, Cl, $OCF_2H$, $CF_3$,

E est CH ou N et

Z est O ou S.

**7.** Agent herbicide, caractérisé en ce qu'il contient un composé de formule (I) ou l'un de ses sels, selon l'une ou plusieurs des revendications 1 à 6, en plus de supports inertes.

**8.** Agent régulateur de la croissance des végétaux, caractérisé en ce qu'il contient un composé de formule (I) ou l'un de ses sels selon au moins l'une des revendications 1 à 6, en plus de supports inertes.

**9.** Utilisation de composés de formule (I) et d'un de ses sels selon l'une ou plusieurs des revendications 1 à 6 en tant qu'herbicides ou régulateurs de la croissance des végétaux.

**10.** Procédé pour préparer un composé de formule (I) selon la revendication (I), caractérisé en ce que :

a) on fait réagir un composé de formule (II)

$R^1$-Y-A-$NR^2$-$SO_2$-N = C = Z (II)

avec un composé de formule (III)

H-$NR^3R^4$ (III)

où, dans les formules (II) et (III), A, Y, Z, $R^1$, $R^2$, $R^3$ et $R^4$ ont les significations données pour la formule (I), ou bien

b) on fait réagir un composé de formule (IV)

$R^1$-Y-A-$NR^2$-$SO_2$-$NH_2$ (IV)

avec un carbamate ou un thiocarbamate de formule (V)

$R^*O$-C-$NR^3R^4$ (V)
‖
Z

où, dans les formules (IV) et (V), $R^1$, $R^2$, $R^3$, $R^4$, A, Y et Z ont les significations données dans la formule (I), et $R^*$ est un radical alkyle en $C_1$-$C_6$, halogénalkyle en $C_1$-$C_4$, phényle, ou encore un radical phényle une ou plusieurs fois substitué par des halogènes ou des radicaux alcoxy en $C_1$-$C_4$ ou nitro, ou bien,

c) on fait réagir un carbamate ou un thiocarbamate de formule (VI) avec un composé ayant la formule (III) mentionnée ci-dessus,

$R^1$-Y-A-$NR^2$-$SO_2$-NH-C-$OR^*$ (VI)
‖
Z

où $R^1$, $R^2$, $R^*$, Y, A et Z ont les significations données ci-dessus, ou bien
d) on fait réagir un composé ayant la formule (VII) ou (VIII)

$R^1$-Y-A-NH-$R^2$ (VII)

$R^1$-Y-A-NH-$R^2$ x HCl (VIII)

avec un composé de formule (IX)

$$\begin{array}{c} ClSO_2\text{-}NH\text{-}\underset{\underset{Z}{\|}}{C}\text{-}NR^3R^4 \end{array} \quad (IX)$$

où, dans les formules (VII) à (IX), A, Y, Z, $R^1$, $R^2$, $R^3$ et $R^4$ ont les significations données ci-dessus.

**Revendications pour l'Etat contractant suivant : ES**

**1.** Utilisation du composé de formule générale (I)

$$R^1\text{-}Y\text{-}A\text{-}NR^2\text{-}SO_2\text{-}NH\text{-}\overset{\overset{Z}{\|}}{C}\text{-}NR^3R^4 \quad (I)$$

dans laquelle

A est un radical hydrocarboné en $C_1$-$C_{10}$, saturé ou insaturé, à chaîne droite ou ramifiée, de préférence un radical de formule $CH_2$, $CH_2CH_2$, CHR, CRR', $CH_2CHR$ ou $CH_2CRR'$, où R et R', indépendamment l'un de l'autre, sont chacun un radical alkyle en $C_1$-$C_4$ ou alcényle en $C_2$-$C_4$.

$R^1$ est un radical alkyle en $C_1$-$C_8$, alcényle en $C_2$-$C_8$, alcynyle en $C_2$-$C_8$, cycloalkyle en $C_3$-$C_8$, cycloalcényle en $C_5$-$C_8$, ou encore l'un des 5 radicaux ci-dessus, substitué une ou plusieurs fois par des halogènes ou par des radicaux qui sont choisis parmi l'ensemble comprenant les substituants alcoxy en $C_1$-$C_6$, alcényloxy en $C_2$-$C_6$, alcynyloxy en $C_2$-$C_6$, alkylthio en $C_1$-$C_6$, alkylsulfinyle en $C_1$-$C_6$, alkylsulfonyle en $C_1$-$C_6$, cycloalkyle en $C_3$-$C_6$, le résidu d'un hétérocycle saturé ayant de 3 à 6 chaînons, avec un atome d'oxygène dans le noyau, ou encore les radicaux furyle, phényle, et les radicaux phényle une ou plusieurs substitués par des substituants choisis parmi l'ensemble comprenant les halogènes et les radicaux alkyle en $C_1$-$C_4$, alcoxy en $C_1$-$C_4$, $CF_3$, (alcoxy en $C_1$-$C_4$)-carbonyle et nitro, ou bien

$R^1$ est le radical phényle ou est un radical phényle une ou plusieurs fois substitué par des radicaux choisis parmi l'ensemble comprenant les halogènes et les radicaux alkyle en $C_1$-$C_4$, alcoxy en $C_1$-$C_4$, $CF_3$, (alcoxy en $C_1$-$C_4$)-carbonyle et nitro, ou bien

$R^1$ est un radical de formule

-CO-$R^5$, -CH$R^6$-COO$R^7$, -CH$R^6$-$CH_2$-COO$R^7$ ou -$CH_2$-CH$R^6$-COO$R^7$,

où, dans les formules, $R^5$ est un radical alkyle en $C_1$-$C_6$, $R^6$ est un hydrogène, un radical alkyle en $C_1$-$C_4$, le radical phényle ou benzyle, et $R^7$ est H ou un radical alkyle en $C_1$-$C_4$ ou le radical phényle ou benzyle,

Y est S ou $SO_2$,

$R^2$ est H ou un radical alkyle en $C_1$-$C_8$, alcényle en $C_2$-$C_8$, alcynyle en $C_2$-$C_8$ ou l'un des trois radicaux ci-dessus, qui sont une ou plusieurs fois substitué par des halogènes ou par des substituants choisis parmi l'ensemble comprenant les radicaux alcoxy en $C_1$-$C_6$, alcényloxy en $C_2$-$C_6$, alcynyloxy en $C_2$-$C_6$, alkylthio en $C_1$-$C_6$, alkylsulfinyle en $C_1$-$C_6$, alkylsulfonyle en $C_1$-$C_6$, (alcoxy en $C_1$-$C_6$)-carbonyle, phénoxycarbonyle, benzyloxycarbonyle et phényle, ou bien encore représente un radical cycloalkyle en $C_3$-$C_8$, lequel est non substitué ou est une ou plusieurs fois substitué par des halogènes ou une ou deux fois par des radicaux alcoxy en $C_1$-$C_4$ ou alkylthio en $C_1$-$C_4$ ; cycloalcényle en $C_5$-$C_8$,

cyclopropylméthyle, époxypropyle, furfuryle, tétrahydrofurfuryle, phénoxy-(alkyle en $C_1$-$C_6$), phényle, ou encore représente l'un des deux derniers radicaux ci-dessus, lequel est substitué sur le noyau phényle par des substituants halogéno, alkyle en $C_1$-$C_4$, alcoxy en $C_1$-$C_4$ ou nitro,

$R^3$ est H ou un radical alkyle en $C_1$-$C_8$, alcényle en $C_2$-$C_8$, alcynyle en $C_2$-$C_8$, ou alcoxy en $C_1$-$C_4$,

$R^4$ est un radical de formule

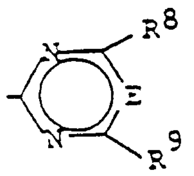 , 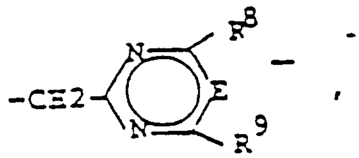 , 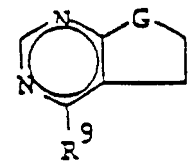 ,

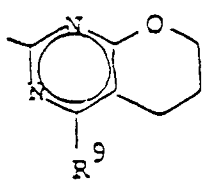 , 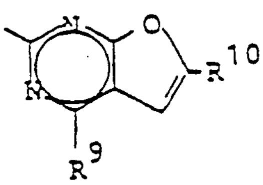 , 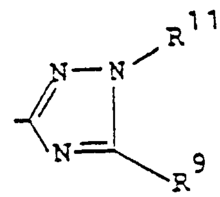 ,

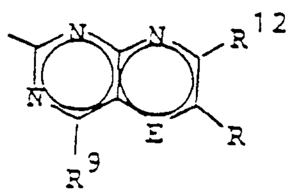 ou 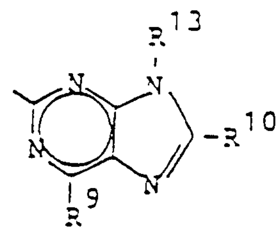

$R^8$ et $R^9$, indépendamment l'un de l'autre, sont chacun H ou un radical halogéno, alkyle en $C_1$-$C_6$, alcoxy en $C_1$-$C_6$, alkylthio en $C_1$-$C_6$, ou encore l'un des trois derniers radicaux ci-dessus, qui sont une ou plusieurs fois substitué par des radicaux halogéno ou une ou deux fois par des substituants alcoxy en $C_1$-$C_4$ ou alkylthio en $C_1$-$C_4$, ou encore un radical $NR^{14}R^{15}$, cycloalkyle en $C_3$-$C_6$, $-OCHR^{16}COOR^{17}$, alcényle en $C_3$-$C_5$, alcynyle en $C_2$-$C_4$, alcényloxy en $C_3$-$C_5$ ou alcynyloxy en $C_3$-$C_5$,

$R^{10}$ est un hydrogène ou un radical alkyle en $C_1$-$C_4$,

$R^{11}$ est un radical alkyle en $C_1$-$C_4$, $-CHF_2$ ou $-CH_2CF_3$,

les radicaux $R^{12}$, indépendamment l'un de l'autre, sont chacun H ou un radical alkyle en $C_1$-$C_4$, alcoxy en $C_1$-$C_4$ ou halogéno,

$R^{13}$ est un hydrogène ou un radical alkyle en $C_1$-$C_4$,$CHF_2$ ou $CH_2CF_3$,

$R^{14}$ et $R^{15}$, indépendamment l'un de l'autre, sont chacun H ou un radical alkyle en $C_1$-$C_4$, alcényle en $C_2$-$C_4$ ou alcynyle en $C_3$-$C_4$,

$R^{16}$ est un hydrogène ou un radical alkyle en $C_1$-$C_4$,

$R^{17}$ est un hydrogène ou un radical alkyle en $C_1$-$C_4$,

E est CH ou N,

G est $CH_2$ ou O, et

Z est O ou S, ainsi que leurs sels, en tant qu'herbicide ou régulateur de croissance des végétaux.

**2.** Utilisation selon la revendication 1, caractérisée en ce que :

$R^1$ est un radical alkyle en $C_1$-$C_4$, un radical alkyle en $C_1$-$C_4$ une ou plusieurs fois substitué par des halogènes ou une ou deux fois substitués par des radicaux alcoxy en $C_1$-$C_4$, alcényloxy en $C_2$-$C_3$, alcynyloxy en $C_2$-$C_3$, alkylthio en $C_1$-$C_4$, alkylsulfinyle en $C_1$-$C_4$, alkylsulfonyle en $C_1$-$C_4$, phényle, ou encore par un radical phényle une à trois fois substitués par des substituants choisis parmi l'ensemble comprenant les halogènes et les radicaux alkyle en $C_1$-$C_4$, alcoxy en $C_1$-$C_4$, $CF_3$, (alcoxy en $C_1$-$C_4$)-

carbonyle et nitro ; ou bien encore cycloalkyle en $C_3$-$C_8$, ou un radical cycloalkyle en $C_3$-$C_8$ une ou plusieurs fois substitué par des halogènes ou une ou deux fois substitués par des substituants alcoxy en $C_1$-$C_4$ ou alkylthio en $C_1$-$C_4$ ; ou bien cycloalcényle en $C_5$-$C_8$, cyclopropylméthyle, époxypropyle, furfuryle, tétrahydrofurfuryle, benzyle, phényle, ou encore un résidu benzyle ou phényle substitué dans le noyau phényle par un ou plusieurs radicaux choisis parmi l'ensemble comprenant les halogènes et les radicaux alkyle en $C_1$-$C_4$, alcoxy en $C_1$-$C_4$, $CF_3$, (alcoxy en $C_1$-$C_4$)-carbonyle et nitro ; ou bien un radical de formule $-COR^5$, $CHR^6$-$COOR^7$, $CHR^6$-$CH_2$-$COOR^7$ ou $CH_2$-$CHR^6$-$COOR^7$,

$R^5$ est un radical alkyle en $C_1$-$C_4$,

$R^6$ est H ou un radical alkyle en $C_1$-$C_4$, benzyle ou phényle,

$R^7$ est H ou radical alkyle en $C_1$-$C_4$, benzyle ou phényle.

**3.** Utilisation selon la revendication 1 ou 2, caractérisée en ce que :

$R^2$ est un radical alkyle en $C_1$-$C_4$, un radical alkyle en $C_1$-$C_4$ une ou plusieurs fois substitué par des halogènes ou une ou deux fois par des substituants alcoxy en $C_1$-$C_4$, alcényloxy en $C_1$-$C_4$, propargyloxy, alkylthio en $C_1$-$C_4$, alkylsulfinyle en $C_1$-$C_4$, alkylsulfonyle en $C_1$-$C_4$, (alcoxy en $C_1$-$C_4$)-carbonyle, phénoxycarbonyle, benzyloxycarbonyle ou phényle, ou encore est un radical cycloalkyle en $C_3$-$C_8$.

**4.** Utilisation selon l'une ou plusieurs des revendications 1 à 3, caractérisée en ce que :

$R^4$ est un radical de formule

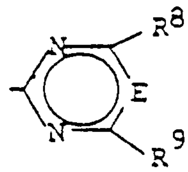

,

et

les radicaux $R^8$ et $R^9$, indépendamment l'un de l'autre, sont chacun un halogène ou un radical alkyle en $C_1$-$C_4$, alcoxy en $C_1$-$C_4$, alkylthio en $C_1$-$C_4$ ou l'un des trois derniers radicaux ci-dessus, qui sont substitués une ou plusieurs fois par des halogènes ou une ou deux fois par des substituants alcoxy ou alkylthio en $C_1$-$C_4$, ou encore un radical $NR^{14}R^{15}$, cycloalkyle en $C_3$-$C_6$, $-OCHR^{16}COOR^{17}$, allyle, propargyle, allyloxy ou propargyloxy,

$R^{14}$ et $R^{15}$, indépendamment l'un de l'autre, sont chacun H ou un radical alkyle en $C_1$-$C_4$,

$R^{16}$ est H ou un radical alkyle en $C_1$-$C_4$,

$R^{17}$ est un radical alkyle en $C_1$-$C_4$, et

E est CH ou N.

**5.** Utilisation selon l'une ou plusieurs des revendications 1 à 4, caractérisée en ce que :

dans une première étape, on estérifie le mono-ester de l'acide citrique avec l'alcool gras ou l'alcool gras alcoxylé, en utilisant éventuellement un catalyseur d'estérification, à une température de 140 à 160 °C tout en éliminant l'eau de la réaction ; dans une deuxième étape, on fait réagir ce dernier en présence d'un catalyseur basique à une température de 80 à 150 °C et sous une pression de 1 à 10 bar avec un oxyde d'alkylène pour donner un produit d'alcoxylation, lequel, dans une troisième étape, est mis à réagir, par addition d'anhydride maléique à une température de 60 à 80 °C pour donner le semi-ester, et, dans une dernière étape, à une température de 60 à 80 °C, avec une solution aqueuse de sulfite de sodium pour donner le sel de l'acide sulfonique correspondant.

**6.** Utilisation selon l'une ou plusieurs des revendications 1 à 5, caractérisée en ce que :

A est un radical de formule $-CH_2-$ ou $-CH_2-CH_2-$,

$R^1$ est un radical alkyle en $C_1$-$C_4$, un radical alkyle en $C_1$-$C_4$ une ou plusieurs fois substitué par des halogènes ou une ou deux fois par des substituants alcoxy en $C_1$-$C_4$ ; cycloalkyle en $C_3$-$C_8$, une ou plusieurs fois substitué par des halogènes, ou non substitué ; benzyle, phényle, ou encore un radical benzyle ou phényle substitué sur le noyau phényle une ou plusieurs fois par des halogènes ou des substituants alkyle en $C_1$-$C_4$, alcoxy en $C_1$-$C_4$, $CF_3$, (alcoxy en $C_1$-$C_4$)-carbonyle ou nitro ; ou bien encore un radical de formule

$-CR^6H-CO_2R^7$

où $R^6$ et $R^7$ sont identiques ou différents et représentent chacun H ou un radical alkyle en $C_1$-$C_4$, phényle ou benzyle,

$R^2$ est un radical alkyle en $C_1$-$C_4$, qui est non substitué ou est substitué une ou plusieurs fois par des halogènes ou par des radicaux (alcoxy en $C_1$-$C_4$)-carbonyle, phényloxycarbonyle, benzyloxycarbonyle ou phényle,

$R^3$ est H ou un radical alkyle en $C_1$-$C_4$ ou allyle, en particulier H,

$R^4$ est un radical de formule

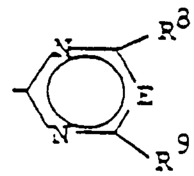 ,

$R^8$ et $R^9$, indépendamment l'un de l'autre, sont chacun un halogène ou un radical alkyle en $C_1$-$C_4$, alcoxy en $C_1$-$C_4$ ou l'un des deux derniers radicaux ci-dessus, lequel est halogéné, en particulier les radicaux $CH_3$, $OCH_3$, $OC_2H_5$, Cl, $OCF_2H$, $CF_3$,

E est CH ou N et

Z est O ou S.

**7.** Procédé pour maîtriser la croissance indésirable des végétaux, caractérisé en ce qu on applique sur les végétaux ou sur l'aire de récolte une quantité efficace d'un composé de formule (I) défini selon l'une ou plusieurs des revendications 1 à 6.

**8.** Procédé de régulation de la croissance des végétaux, caractérisé en ce qu'il contient un composé de formule (I) selon l'une ou plusieurs des revendications 1 à 6, en plus de supports inertes.

**9.** Procédé pour préparer un composé ayant la formule (I) définie dans la revendication 1, caractérisé en ce que :

a) on fait réagir un composé de formule (II)

$R^1$-Y-A-$NR^2$-$SO_2$-N = C = Z (II)

avec un composé de formule (III)

H-$NR^3R^4$ (III)

où, dans les formules (II) et (III), A, Y, Z, $R^1$, $R^2$, $R^3$ et $R^4$ ont les significations données pour la formule (I), ou bien

b) on fait réagir un composé de formule (IV)

$R^1$-Y-A-$NR^2$-$SO_2$-$NH_2$ (IV)

avec un carbamate ou un thiocarbamate de formule (V)

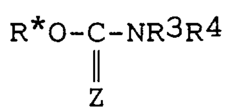

(V)

où, dans les formules (IV) et (V), $R^1$, $R^2$, $R^3$, $R^4$, A, Y et Z ont les significations données dans la formule (I), et R* est un radical alkyle en $C_1$-$C_6$, halogénalkyle en $C_1$-$C_4$, phényle, ou encore un radical phényle une ou plusieurs fois substitué par des halogènes ou des radicaux alcoxy en $C_1$-$C_4$

ou nitro, ou bien,
c) on fait réagir un carbamate ou un thiocarbamate de formule (VI) avec un composé ayant la formule (III) mentionnée ci-dessus,

$$R^1\text{-}Y\text{-}A\text{-}NR^2\text{-}SO_2\text{-}NH\text{-}\underset{\substack{\| \\ Z}}{C}\text{-}OR^* \qquad (VI)$$

où $R^1$, $R^2$, $R^*$, Y, A et Z ont les significations données ci-dessus, ou bien
d) on fait réagir un composé ayant la formule (VII) ou (VIII)

$R^1$-Y-A-NH-$R^2$ (VII)

$R^1$-Y-A-NH-$R^2$ x HCl (VIII)

avec un composé de formule (IX)

$$ClSO_2\text{-}NH\text{-}\underset{\substack{\| \\ Z}}{C}\text{-}NR^3R^4 \qquad (IX)$$

où, dans les formules (VII) à (IX), A, Y, Z, $R^1$, $R^2$, $R^3$ et $R^4$ ont les significations données ci-dessus.